# DEMANDE DE BREVET EUROPEEN

(11) **EP 4 050 110 A1**
(43) Date de publication de la demande: **31.08.2022**
(21) Numéro de dépôt: 22158634.0
(22) Date de dépôt: 24.02.2022
(51) Int. Cl.: C12Q 1/6806, C12Q 1/6886

(54) **MÉTHODE DE CARACTERISATION D'UNE TUMEUR À L'AIDE D'UN SÉQUENÇAGE CIBLÉ**

(30) Priorité: 25.02.2021 BE 202105136
(71) Demandeur: OncoDNA, 6041 Gosselies (BE)
(72) Inventeur: Laes, Jean-François, 1400 Nivelles (BE); Liénard, Maxime, 5060 Auvelais (BE); Zaag, Rim, 91000 Evry (FR)
(74) Mandataire: Calysta NV

(57) **Abrégé**

Méthode de caractérisation d'une tumeur par un séquençage ciblé comprenant une récolte d'ADN génomique d'une tumeur, un séquençage des fragments d'ADN et une caractérisation de lα tumeur; le séquençage et lα caractérisation comprennent une étape d'hybridation de fragments d'ADN à l'aide d'une pluralité de sondes d'hybridation d'ADN double brin pour donner des fragments hybridés et une étape d'enrichissement uniforme de séquences d'ADN simples et complexes desdits fragments d'ADN hybridés; lα caractérisation de lα tumeur étant définie par un séquençage de séquences d'ADN cibles de manière à identifier des aberrations génétiques de lα tumeur.

## Description

La présente invention se rapporte à une méthode de caractérisation d'une tumeur par un séquençage ciblé à partir d'un échantillon d'un patient atteint d'un cancer, comprenant les étapes de :
- une récolte d'ADN génomique à partir dudit échantillon comprenant au moins une cellule cancéreuse,
- une préparation d'une bibliothèque de séquençage à partir dudit échantillon comprenant les étapes successives de (i) une fragmentation dudit ADN génomique générant des fragments d'ADN, (ii) un ajout d'une série d'étiquettes aux extrémités desdits fragments d'ADN, (iii) une première amplification desdits fragments d'ADN à l'aide d'amorces ayant une séquence complémentaire à une séquence desdites étiquettes formant un premier mélange comprenant des fragments d'ADN amplifiés,
- un blocage desdits fragments d'ADN amplifiés dudit premier mélange à l'aide d'une série de bloqueurs ayant une séquence complémentaire à au moins une partie de la séquence des étiquettes pour former des fragments d'ADN bloqués, et
- un séquençage et une caractérisation de la tumeur.

Un état pathologique chez un patient est généralement traité avec un schéma thérapeutique ou une thérapie sélectionnée en fonction de critères cliniques; c'est-à-dire qu'une thérapie ou un traitement est sélectionné pour un patient sur base de la détermination du fait que le patient a été diagnostiqué avec une maladie particulière (lequel diagnostic a été établi à partir d'essais diagnostiques classiques). Bien que les mécanismes moléculaires sous-jacents divers états pathologiques, en particulier divers cancers, aient fait l'objet d'études pendant des années, l'application spécifique du profil moléculaire d'un individu malade pour déterminer un schéma thérapeutique et une thérapie ciblée à cet individu n'a pas été largement poursuivie.

Un cancer est une maladie caractérisée par des aberrations génétiques multiples qui peuvent être différentes en fonction du type de cancer, et qui peuvent même être très différentes pour un même type de cancer chez différents patients. Par les termes « type de cancer», on entend au sens de la présente invention un cancer qui atteint un même tissu ou un même organe.

Sans être exhaustif, une aberration génétique peut comprendre une mutation ponctuelle de nucléotide, un polymorphisme d'un seul nucléotide, une insertion, une délétion, une translocation, une variation du nombre de copie d'un gène ou d'une séquence d'ADN spécifique, une délétion d'une copie ou de deux copies d'un gène, un phénotype de défaut de recombinaison homologue HRD, une charge de mutation tumorale spécifique, une instabilité microsatellitaire, ou encore un déséquilibre allélique des télomères. La tumeur peut être caractérisée par une ou plusieurs aberrations génétiques différentes. Sachant que d'un patient à l'autre, il peut y avoir énormément d'hétérogénéité mutationnelle pour un même type de cancer, l'efficacité d'un traitement contre le cancer peut être très différente en fonction du patient. En effet, un traitement contre le cancer est généralement spécifique de certaines cibles moléculaires exprimées dans la tumeur. Pour qu'un traitement contre le cancer soit efficace, il faut que ces cibles moléculaires soient bien exprimées dans la tumeur. Un profil mutationnel spécifique de la tumeur entraîne l'activation et/ou l'inhibition de certaines voies de signalisation cellulaire et/ou de certaines voies métaboliques pouvant faire en sorte que la tumeur exprime ou non les cibles moléculaires spécifiques du traitement contre le cancer. En conséquence, la tumeur peut être sensible ou résistante audit traitement contre le cancer. Il est donc important de pouvoir identifier de manière spécifique le profil moléculaire et le profil génétique de chaque tumeur avec la plus grande précision possible de manière à pouvoir proposer au patient un traitement qui soit le mieux adapté à son profil moléculaire et génétique. En plus du profil génétique de la tumeur déterminé par une identification d'aberrations génétiques, il faut entendre au sens de la présente invention par profil moléculaire de la tumeur, une détermination de l'expression de marqueurs moléculaires de la tumeur qui peuvent comprendre des ARNs, et/ou des microARNs, et/ou des protéines.

Un séquençage ciblé est une technique de séquençage qui permet de sélectionner de manière spécifique des régions génomiques d'intérêts préalablement à l'étape de séquençage. Un séquençage ciblé permet d'éviter de devoir séquencer tout le génome (séquençage en génome complet) ou tout l'exome (séquençage en exome complet). De manière à effectuer un séquençage ciblé de régions génomiques spécifiques, ces régions génomiques doivent être capturées et enrichies de manière spécifique avant d'être séquencées.

Une méthode de caractérisation d'une tumeur par un séquençage ciblé à partir d'un échantillon d'un patient atteint d'un cancer est connue par exemple du document EP2721181 qui décrit une méthode de séquençage ciblé pour caractériser une tumeur. Selon ce document, les sondes d'hybridation sont des sondes conventionnelles, ce qui limite le nombre de caractéristiques tumorales pouvant être identifiées étant donné le nombre de faux positifs et faux négatifs générés par ce type de méthode de séquençage ciblé. En effet, des sondes d'hybridation conventionnelles vont enrichir certaines séquences d'ADN de la tumeur moins bien que d'autres, ce qui limite la quantité d'information générée avec une couverture de séquençage donnée et ce qui génère des faux négatifs et des faux positifs dans l'identification d'aberrations génétiques.

Le document EP2981624 concerne des méthodes de séquençage ciblé impliquées dans l'évaluation d'échantillons (par exemple, des cellules cancéreuses ou des acides nucléiques dérivés de celles-ci) pour un phénotype de défaut de recombinaison homologue HRD (par exemple, une signature HRD) sur la base de la détection d'aberrations chromosomiques particulières (CA). Un phénotype HRD est caractérisé par une déficience du mécanisme de réparation des cassures double-brin de l'ADN par recombinaison homologue. Ce document fournit des procédés et des matériaux pour détecter des régions CA afin de déterminer si une cellule (par exemple, une cellule cancéreuse) présente un phénotype HRD. Une cellule présentant un phénotype HRD sera donc plus sensible à des traitements assurant des cassures de l'ADN. Ce document fournit également des matériaux et des méthodes pour identifier les patients cancéreux susceptibles de répondre à un régime de traitement du cancer particulier sur la base de la présence, de l'absence ou de la gravité d'un HRD.

Le document EP2659005 concerne des procédés et un système de profilage moléculaire entre autres par un séquençage ciblé, utilisant les résultats du profilage moléculaire pour identifier des traitements ciblés pour des individus. Le procédé d'identification d'un traitement candidat pour un sujet qui en a besoin, comprend: (a) la détermination d'un profil moléculaire pour un ou plusieurs échantillons du sujet à l'aide d'un panel de gène ou de produits géniques, (b) comparer le profil moléculaire du sujet à un profil moléculaire de référence pour identifier quels membres du panel sont exprimés de manière différente entre le ou les échantillons et la référence; et (c) identifier un traitement qui est associé à un ou plusieurs membres du panel, où pour ce faire ledit un ou lesdits plusieurs membres du panel sont exprimés de manière différente entre le ou les échantillons et la référence.

Le document US2020118644 concerne l'utilisation d'un séquençage de nouvelle génération pour déterminer l'état d'instabilité des microsatellites (MSI). Ce document présente des techniques pour déterminer directement l'instabilité des microsatellites à partir de cartographies de régions microsatellitaires pour des locus spécifiques dans le génome. Les techniques fournissent un processus automatisé pour les tests MSI et la prédiction de l'état MSI via un processus d'apprentissage automatique supervisé.

Malheureusement, de telles méthodes telle que décrites ci-dessus présentent encore de nombreux inconvénients. En effet, il existe une demande grandissante pour identifier de manière fiable un nombre important d'aberrations génétiques afin d'améliorer la caractérisation des tumeurs. A ce jour, pour identifier un grand nombre de caractéristiques tumorales à l'aide d'un séquençage, l'état de la technique suggère de recourir à un séquençage en génome complet, qui permet, par définition, d'avoir une vue globale sur les aberrations génétiques d'une tumeur mais qui est aujourd'hui encore trop coûteux et trop lent pour être utilisé comme diagnostic médical de manière quotidienne. Une alternative au séquençage génome complet est de réaliser un séquençage ciblé de certaines régions génomiques prédéterminées. Cependant, à ce jour, ce type de séquençage ciblé ne permet pas d'identifier suffisamment d'aberrations génétiques d'une tumeur pour rendre compte de manière précise de la complexité génétique d'un échantillon tumoral avec une certitude acceptable. En effet, les techniques conventionnelles de séquençage ciblé séquencent certaines régions génomiques de la tumeur moins bien que d'autres, ce qui rend le séquençage non-uniforme et ce qui limite la quantité d'information générée avec une couverture de séquençage donnée. La non-uniformité du séquençage susdite et la quantité d'information limitée de séquençage générée avec une couverture de séquençage donnée créent par ailleurs un nombre important de faux positifs et de faux négatifs dans l'identification d'aberrations génétiques, ce qui ne permet de caractériser la tumeur de manière fiable qu'avec un nombre insuffisant d'aberrations génétiques. En effet, si la quantité d'information de séquençage générée est limitée par la non-uniformité du séquençage, il en résultera une faible puissance statistique dans l'identification d'aberrations génétiques, ce qui entraînera un nombre conséquent de faux positifs et de faux négatifs. Une caractérisation d'un nombre suffisant d'aberrations génétiques de la tumeur de manière fiable est importante pour établir au mieux un traitement adapté pour le patient. En effet, étant donné la lourdeur des traitements contre le cancer pour le patient, comme par exemple lors de chimiothérapies, immunothérapies, ... tant au niveau de la durée du traitement qu'au niveau des effets secondaires, il est très important d'éviter des administrations de thérapies sans résultats. En effet, en oncologie, il existe encore de nombreux cas où le thérapeute administre un traitement et en fonction de la réponse du patient décide de poursuivre le traitement ou d'administrer une autre thérapie. Toutefois de tels traitements sont très lourds de conséquence sur la santé générale du patient et lorsqu'ils ne donnent pas de résultats, parfois, un temps précieux est perdu. Aussi, une recommandation d'un traitement de chimiothérapie ou d'immunothérapie identifié sur base d'un faux positif ou d'un faux négatif peut ainsi être très pénalisant pour le patient. Il existe donc un besoin de disposer de méthodes fiables de caractérisation de la tumeur dans lesquelles le nombre de faux positifs et de faux négatifs dans le séquençage ciblé d'une tumeur est limité au maximum tout en étant accessible et pouvant être effectué dans un délai acceptable.

La présente invention a pour but de pallier ces inconvénients en procurant une méthode moins coûteuse, plus rapide et plus fiable par rapport aux techniques conventionnelles de caractérisation de la tumeur. Pour ceci, la présente invention procure une méthode de caractérisation d'une tumeur par un séquençage ciblé à partir d'un échantillon d'un patient atteint d'un cancer dans laquelle ledit séquençage et ladite caractérisation de la tumeur comprennent les étapes de :
- une collecte d'une pluralité de sondes d'hybridation d'ADN double brin spécifiques à une pluralité desdits fragments d'ADN bloqués,
- une dénaturation desdites sondes d'hybridation d'ADN double brin pour former une pluralité de sondes d'hybridation d'ADN dénaturées spécifiques à ladite pluralité desdits fragments d'ADN bloqués,
- une hybridation de la pluralité desdits fragments d'ADN bloqués par la pluralité de sondes d'hybridation d'ADN dénaturées pour former un deuxième mélange comprenant des fragments d'ADN hybridés et des fragments d'ADN non-hybridés à la pluralité de sondes d'hybridation d'ADN dénaturées,
- un enrichissement uniforme et simultané du deuxième mélange en fragments d'ADN contenant des séquences simples et en fragments d'ADN contenant des séquences complexes desdits fragments d'ADN hybridés à la pluralité de sondes d'hybridation d'ADN dénaturées par une capture desdits fragments d'ADN hybridés pour former un milieu enrichi en lesdits fragments d'ADN hybridés à la pluralité de sondes d'hybridation d'ADN dénaturées,
- un lavage et une récupération desdits fragments d'ADN hybridés à la pluralité de sondes d'hybridation d'ADN dénaturées pour former un milieu enrichi avec des séquences d'ADN cibles,
- une deuxième amplification desdites séquences d'ADN cibles à l'aide d'amorces ayant une séquence complémentaire à une séquence desdites étiquettes donnant des séquences d'ADN cibles à séquencer,
- ladite caractérisation de la tumeur étant une caractérisation par un séquençage des fragments d'ADN contenant des séquences simples et des fragments d'ADN contenant des séquences complexes desdites séquences d'ADN cibles à séquencer de manière à identifier des aberrations génétiques de la tumeur.

Selon la présente invention, les sondes d'hybridation d'ADN double brin spécifiques permettent d'enrichir de manière uniforme à la fois des fragments d'ADN contenant des séquences simples et des fragments d'ADN contenant des séquences complexes car lorsque les sondes d'hybridation d'ADN double brin spécifiques sont dénaturées, les deux brins d'ADN de chaque sonde d'hybridation d'ADN double brin spécifiques (les sondes d'hybridation d'ADN dénaturées) permettent de s'hybrider sur le brin sens de la région génomique cible et le brin antisens audit brin sens de la région génomique cible. Une hybridation des sondes d'hybridation d'ADN dénaturées sur le brin sens et antisens de la région génomique cible permet une capture plus efficace, et donc un enrichissement plus uniforme, des différents types de fragments d'ADN comme les fragments d'ADN contenant des séquences simples et les fragments d'ADN contenant des séquences complexes. Le séquençage ciblé subséquent à cette étape d'enrichissement permet alors de séquencer de manière bien plus uniforme des fragments d'ADN contenant des séquences simples et des fragments d'ADN contenant des séquences complexes puisque l'enrichissement est plus uniforme. Ceci permet ensuite d'identifier plus de caractéristiques tumorales, et assure donc, *in fine,* une caractérisation plus complète de la tumeur et ce, à coût réduit.

En effet, le fait d'avoir un enrichissement uniforme de séquences d'ADN simples et complexes desdits fragments d'ADN hybridés à pluralité de sondes d'hybridation d'ADN dénaturées permet un séquençage uniforme et optimal des séquences simples et complexes desdites séquences d'ADN cibles à séquencer. Ce séquençage uniforme permet de générer bien plus d'information de séquençage pour une couverture de séquençage donnée. Le fait de générer un maximum d'information de séquençage pour une couverture de séquençage donnée permet de limiter le nombre de faux positifs et de faux négatifs obtenus lors de l'identification des aberrations génétiques de la tumeur car l'identification des aberrations génétiques reposera sur un plus grand nombre de données de séquençage et donc sur une plus grande puissance statistique, ce qui rend la méthode selon la présente invention moins coûteuse et plus fiable.

Bien que des techniques d'enrichissement de régions génomiques cibles avec des sondes d'hybridation ADN double brin soient connues, (voir par exemple les documents WO2019/008172A1, US2014/243232A1, US2020017907A1), les techniques basées sur des sondes d'hybridation ADN double brin ne sont tantôt pas appliquées dans le domaine de l'oncologie pour identifier un large panel d'aberrations génétiques voire pas appliquées pour des séquences non codantes et/ou complexes. Or, pour identifier un large panel d'aberrations génétiques de la tumeur, il est nécessaire de pouvoir enrichir et séquencer des régions génomiques complexes car celles-ci contiennent une grande quantité d'information qui permet de mieux identifier ces aberrations génétiques tumorales et donc de fournir une caractérisation plus complète de la tumeur. Or, avant de pouvoir enrichir et séquencer ces régions génomiques complexes, il faut pouvoir les identifier de manière telles que ces régions génomiques complexes soient représentatives d'aberrations génétiques multiples. Toutefois, les échantillons tumoraux sont caractérisés par une quantité importante de tissus nécrotiques et/ou de cellules en apoptose qui réduisent la qualité de l'ADN extrait et qui en limitent ainsi les possibilités de séquençage. De plus, en raison d'un profil mutationnel spécifique à chaque tumeur, la séquence des régions génomiques d'une tumeur peut être très variable en fonction de la tumeur analysée. Par conséquent, il n'y avait aucune raison qu'on puisse s'attendre à avoir une couverture de séquençage plus uniforme à l'aide de sondes d'hybridation d'ADN double brin pour séquencer à la fois des régions génomiques contenant des séquences simples et des séquences complexes de la tumeur. En effet, ces régions génomiques peuvent présenter des profils mutationnels différents en fonction de la tumeur analysée et il est donc surprenant de pouvoir identifier avec suffisamment de spécificité et de robustesse une pluralité d'aberrations génétiques tumorales assurant une caractérisation plus complète de la tumeur et ce, quel que soit le type de tumeur et d'échantillon tumoral analysé.

Au sens de la présente invention, le terme « sondes d'hybridation d'ADN double brin spécifiques » désigne par exemple des sondes spécifiques présentant une séquence complémentaire à une séquence choisie dans des régions génomiques cibles. Au sens de la présente invention, les sondes d'hybridation d'ADN double brin spécifiques comprennent donc au moins une sonde spécifique d'un fragment d'ADN contenant une séquence simple et au moins une sonde spécifique d'un fragment d'ADN contenant une séquence complexe. De préférence, les sondes d'hybridation d'ADN double brin spécifiques comprennent au moins 50, plus particulièrement 100, avantageusement au moins 500 sondes spécifiques d'un ou de plusieurs fragment d'ADN contenant une séquence simple et au moins 50, plus particulièrement 100, avantageusement au moins 500 sondes spécifiques d'un ou de plusieurs fragment d'ADN contenant une séquence complexe, voire un nombre total de sondes d'hybridation d'ADN double brin spécifiques compris entre 100 et 20000, plus particulièrement entre 200 et 10000, de manière favorable entre 500 et 5000.

Au sens de la présente invention, le terme « région génomique complexe » ou « séquences complexes » désigne par exemple
- des régions riches en base nucléotidique guanine et cytosine comme c'est souvent le cas pour les régions promotrices et le premier exon d'un gène, typiquement des régions définies par des séquences nucléotidiques présentant un pourcentage de base guanine et cytosine par rapport au nombre total des bases nucléotidiques formant les séquences nucléotidiques égale ou supérieur à 60%, préférentiellement égale ou supérieur à 70%, de manière favorable égale ou supérieur à 80%, et/ou
- des régions riches en base nucléotidique adénine et thymine, typiquement des régions définies par des séquences présentant un pourcentage de base adénine et thymine par rapport au nombre total des bases nucléotidiques formant les séquences nucléotidiques égale ou supérieur à 60%, préférentiellement égale ou supérieur à 70%, de manière favorable égale ou supérieur à 80%, et/ou
- des régions telles que des centromères ou télomères, et/ou
- des régions présentant une structure en épingle à cheveux où une séquence contient deux répétitions de nucléotides inversés qui sont séparés par au moins trois nucléotides, et/ou
- des séquences répétées définies par une répétition de deux ou trois nucléotides.

Comme on peut le constater, la présente invention permet donc de caractériser une tumeur par un séquençage ciblé à partir d'un échantillon d'un patient atteint d'un cancer. Les étapes d'hybridation et d'enrichissement du séquençage ciblé sont réalisées à l'aide d'une pluralité de sondes d'hybridation d'ADN dénaturées provenant de la dénaturation d'une pluralité de sondes d'hybridation d'ADN double brin, qui permet un enrichissement plus uniforme des fragments d'ADN cibles contenant des séquences simples et des fragments d'ADN cibles contenant des séquences complexes. Cet enrichissement uniforme de l'ensemble des fragments d'ADN cibles permet à la fois un séquençage très précis de l'ensemble de ces fragments d'ADN, une réduction du nombre de faux positifs et de faux négatifs et un maintien d'une couverture moyenne de séquençage suffisante et une réduction conséquente des coûts de séquençage. Le séquençage uniforme de fragments d'ADN cibles de séquences simples et complexes selon la présente invention permet d'identifier un grand nombre d'aberrations génétiques de la tumeur car de nombreuses aberrations génétiques sont localisées dans des régions contenant des séquences simples, mais également dans des régions génomiques complexes comme par exemple des régions riches en base nucléotidique guanine et cytosine et/ou des centromères et/ou des télomères et/ou des régions riches en séquences répétées, garantissant de la sorte une caractérisation plus complète de la tumeur.

Selon la présente invention, on entend par les termes « amorce ayant une séquence complémentaire à une séquence desdites étiquettes », une amorce qui présente une séquence présentant une complémentarité de séquence à une séquence d'une ou plusieurs desdites étiquettes d'au moins 80%, préférentiellement au moins 90%, encore plus préférentiellement au moins 92%, 93%, 94%, 95%, 96%, 97%, 98%, 99%, 100%.

Selon la présente invention, on entend par les termes « bloqueur ayant une séquence complémentaire à au moins une partie de la séquence des étiquettes», un bloqueur ayant une séquence telle que la complémentarité de séquence à la séquence des étiquettes est d'au moins 50%, préférentiellement, au moins 80%, plus préférentiellement au moins 90%, de manière avantageuse au moins 92%, 93%, 94%, 95%, 96%, 97%, 98%, 99%, 100%.

Selon la présente invention, on entend par les termes «un enrichissement uniforme (ou plus uniforme) et simultané du deuxième mélange en fragments d'ADN contenant des séquences simples et en fragments d'ADN contenant des séquences complexes desdits fragments d'ADN hybridés à la pluralité de sondes d'hybridation d'ADN dénaturées», un enrichissement de séquences d'ADN simples et complexes ayant une uniformité d'enrichissement égale ou supérieure à 80%, plus préférentiellement égale ou supérieure à 85%, encore plus préférentiellement égale ou supérieure à 90%, de manière favorable égale ou supérieure à 91%, 92%, 93%, 94%, 95%, 96%, 97%, 98%, 99%. Plus particulièrement, on entend que l'enrichissement d'une première séquence simple sera par exemple similaire à l'enrichissement d'une deuxième séquence complexe à 20% près, plus préférentiellement à 15% près, plus particulièrement à 10% ou mieux à 9%, à 8%, à 7%, à 6%, à 5%, à 4%, à 3%, à 2% à 1% près.

Plus particulièrement, on peut aussi définir l'enrichissement uniforme (ou plus uniforme) et simultané de séquences d'ADN simples et complexes de la manière suivante :
si le niveau d'enrichissement de la première séquence simple est X1, alors, le niveau d'enrichissement de la deuxième séquence complexe est Y1 tel que Y1 est compris entre 0,8^{∗}X1 et 1,2^{∗}X1, de préférence compris entre 0,85^{∗}X1 et 1,15^{∗}X1, plus particulièrement entre 0,9^{∗}X1 et 1,1^{∗}X1 ou mieux entre 0,91^{∗}X1 et 1,09^{∗}X1, entre 0,92^{∗}X1 et 1,08^{∗}X1, entre 0,93^{∗}X1 et 1,07^{∗}X1, entre 0,94^{∗}X1 et 1,06^{∗}X1, entre 0,95^{∗}X1 et 1,05^{∗}X1, entre 0,96^{∗}X1 et 1,04^{∗}X1, entre 0,97^{∗}X1 et 1,03^{∗}X1, entre 0,98^{∗}X1 et 1,02^{∗}X1, entre 0,99^{∗}X1 et 1,01^{∗}X1, de telle manière que le niveau d'enrichissement uniforme et simultané de séquences d'ADN simples et de séquences d'ADN complexes X1/Y1 soit compris entre 0,8 et 1,2, de préférence entre 0,85 et 1,15, plus particulièrement entre 0,9 et 1,1, de manière favorable entre 0,91 et 1,09, entre 0,92 et 1,08, entre 0,93 et 1,07, entre 0,94 et 1,06, entre 0,95 et 1,05, entre 0,96 et 1,04, entre 0,97 et 1,03 entre 0,98 et 1,02, entre 0,99 et 1,01.

On entend également qu'un niveau d'enrichissement donné de chaque séquence d'ADN simple et/ou complexe est égal à la moyenne arithmétique +/- 20% du niveau d'enrichissement de toutes les séquences d'ADN simples et/ou complexes, plus préférentiellement est égal à la moyenne arithmétique +/- 15%, ou mieux +/- 10%, +/- 9%, +/- 8%, +/- 7%, +/- 6%, +/- 5%, +/- 4%, +/- 3%, +/- 2%, +/- 1% du niveau d'enrichissement de toutes les séquences d'ADN simples et/ou complexes.

De manière avantageuse, l'enrichissement de séquences d'ADN simples et complexes est réalisé de manière simultanée dans les mêmes conditions.

Par ailleurs, dans la méthode de caractérisation selon la présente invention, après l'hybridation, le lavage et la récupération desdits fragments d'ADN hybridés permettent d'éliminer une quantité importante d'impuretés et assurent donc une meilleure pureté du milieu enrichi comprenant des séquences d'ADN cibles, ce qui permet une deuxième amplification optimale desdites séquences d'ADN cibles et donc aussi un séquençage optimal desdites séquences d'ADN cibles à séquencer.

Avantageusement, dans la méthode de caractérisation selon la présente invention, la récolte d'ADN génomique à partir d'un échantillon comprenant au moins une cellule cancéreuse comprend une ou plusieurs des étapes suivantes :
- une réception d'un échantillon formé soit d'un bloc comprenant un morceau de tumeur extrait par une biopsie fixé au formol et inclus dans la paraffine (bloc FFPE) soit de lames de tissus tumoraux déjà coupées ;
- si l'échantillon est formé d'un bloc, un découpage du bloc en lames d'une épaisseur comprise entre 4 et 15 µm;
- une coloration de la première et dernière lame avec de l'hematoxiline éosine (lames H&E) ;
- une identification à partir de la première lame, éventuellement par inspection visuelle, d'une zone de la lame comprenant suffisamment de cellules tumorales, peu ou pas de nécrose et une infiltration lymphocytaire inférieure à 21% ;
- sur cette première lame colorée, un marquage d'une région comprenant le plus de cellules tumorales;
- un report du marquage de la première lame sur les lames non colorée pour marquer la même région de la tumeur, mais sur une tranche différente ;
- un grattage des cellules de ces régions marquées (étape dite de macrodissection) ;
- une extraction de l'ADN de ces lames pour former l'ADN génomique à caractériser ;
- en parallèle, une quantification de l'ADN à l'aide d'un spectrophotomètre à partir d'un échantillon de l'ADN génomique à caractériser;
- en parallèle, une qualification de l'ADN par migration sur gel à partir d'un échantillon de l'ADN génomique à caractériser.

Avantageusement, dans la méthode de caractérisation selon la présente invention, ledit échantillon comprenant au moins une cellule cancéreuse est un échantillon sanguin ou un échantillon d'urine. En effet, lorsque l'ADN tumoral se retrouve sous forme d'ADN circulant dans un échantillon sanguin ou un échantillon d'urine, la méthode de caractérisation selon la présente invention permet de caractériser la tumeur sans intervention invasive pour le patient tout en apportant suffisamment de précision pour caractériser les aberrations génétiques de la tumeur.

Préférentiellement, dans la méthode de caractérisation selon la présente invention, lesdits fragments d'ADN contenant des séquences complexes desdites séquences d'ADN cibles à séquencer étant des séquences présentant un pourcentage de base nucléotidique guanine et cytosine égal ou supérieur à 60%, préférentiellement égal ou supérieur à 70%, de manière favorable égale ou supérieur à 80% ou des séquences répétitives ou des séquences inversées. En effet, ces fragments d'ADN riche en guanine et cytosine sont plus difficiles à dénaturer et sont plus difficiles à enrichir lors de l'étape d'enrichissement d'un séquençage ciblé et ce d'autant plus que les fragments d'ADN contenant des séquences simples et les fragments d'ADN contenant des séquences complexes sont séquencées typiquement durant la même étape, à savoir dans des conditions qui représentent le meilleur compromis tant pour les séquences simples que pour les séquences complexes

Avantageusement, dans la méthode de caractérisation selon la présente invention, lesdits fragments d'ADN hybridés à ladite pluralité de sondes d'hybridation d'ADN dénaturées présentent une séquence dont au moins une partie est une séquence codante desdits fragments d'ADN contenant les séquences simples et/ou desdits fragments d'ADN contenant les séquences complexes et/ou une séquence dont au moins une partie est une séquence non-codante desdits fragments d'ADN contenant les séquences simples et/ou desdits fragments d'ADN contenant les séquences complexes. Les séquences non-codantes au sens de l'invention sont des séquences non-codantes d'un gène ou des séquences non-codantes à en dehors du gène dans une proximité relative de telle manière qu'elles sont accessibles au séquençage. En effet, les aberrations génétiques qui caractérisent une tumeur se situent à de multiples positions dans le génome au sein d'une séquence codante ou non-codante d'un gène ou bien d'une séquence en dehors d'un gène. Il est donc avantageux de cibler simultanément un ensemble de fragments d'ADN représentatif d'une pluralité d'aberrations génétiques possibles ou d'une absence d'aberrations génétiques possibles en hybridant ces fragments d'ADN à pluralité de sondes d'hybridation d'ADN dénaturées spécifiques, de manière à caractériser la tumeur de façon la plus complète possible. Lors du séquençage ciblé de la présente invention, les fragments d'ADN contenant des séquences simples et les fragments d'ADN contenant des séquences complexes sont séquencés simultanément. La pluralité de sondes d'hybridation d'ADN dénaturées spécifiques provenant des sondes d'hybridation d'ADN double brin spécifiques, cible un ensemble de fragments d'ADN qui permet l'identification d'une pluralité d'aberrations génétiques possibles ou d'une absence d'aberrations génétiques. Ceci permet de pallier l'hétérogénéité du profil mutationnel d'une tumeur à l'autre et d'être extrêmement polyvalent car les aberrations génétiques ou l'absence d'aberrations génétiques qui sont identifiées dans la méthode de caractérisation selon la présente invention peuvent donc être différentes d'un type de tumeur à l'autre et/ou pour différentes tumeurs d'un même type de tumeur.

De manière préférée, lesdites séquences d'ADN cibles à séquencer contiennent des aberrations génétiques comprenant une série de polymorphisme d'un seul nucléotide d'une ou de plusieurs séquences d'ADN par rapport à une ou plusieurs séquences d'ADN correspondante d'un génome de référence, ladite série de polymorphisme d'un seul nucléotide comprenant préférentiellement au moins 50, plus préférentiellement au moins 500, encore plus préférentiellement au moins 1500, de manière favorable au moins 3000 polymorphismes d'un seul nucléotide, lesdits polymorphismes d'un seul nucléotide étant localisés, en moyenne sur tout le génome, toutes les 0,5 à 50 mégabases, préférentiellement toutes les 5 à 25 mégabases, plus préférentiellement toutes les 10 à 20 mégabases, de manière favorable toutes les 14 à 15 mégabases.

Avantageusement, le génome de référence est un génome de l'humain. De manière préférée, la séquence nucléotidique du génome de référence est obtenue à l'aide de banques de données publiques comme hg19 (par exemple: grch37) de NCBI: https://www.ncbi.nlm.nih.gov/seorch/all/?term=arch37.p13. Avantageusement, le génome de référence peut également être un génome provenant d'un échantillon sain comprenant des cellules saines (non-tumorales) dudit patient atteint d'un cancer dont on a prélevé ledit échantillon comprenant au moins une cellule cancéreuse. Les aberrations génétiques de la tumeur dudit patient peuvent donc être identifiées soit en comparaison par rapport à un génome de référence comme par exemple le génome de l'humain provenant d'une base de données publique comme par exemple hg19, soit en comparaison par rapport au génome provenant dudit échantillon sain comprenant des cellules saines (non-tumorales) provenant dudit patient atteint d'un cancer.

De manière préférentielle, dans la méthode de caractérisation d'une tumeur par un séquençage ciblé selon la présente invention, lesdites séquences d'ADN cibles à séquencer contiennent des aberrations génétiques comprenant une délétion d'une copie ou de deux copies d'un gène par rapport audit génome de référence. Cette aberration génétique permet entre autres de déterminer la perte d'hétérozygotie de certains gènes, ce qui représente un mécanisme important dans le développement tumoral, notamment lorsque la perte d'hétérozygotie est présente pour des gènes suppresseurs de tumeur.

Avantageusement, dans la méthode de caractérisation selon la présente invention, lesdites séquences d'ADN cibles à séquencer contiennent des aberrations génétiques comprenant une variation du nombre de copies par rapport au nombre de copies dudit génome de référence associée à au moins 2 régions génomiques, préférentiellement au moins 50 régions génomiques, plus préférentiellement au moins 100 régions génomiques, de manière favorable au moins 250 régions génomiques et à maximum 5000 régions génomiques, de manière préférée maximum 2000 régions génomiques, de manière favorable maximum 1000 régions génomiques réparties de manière aléatoire dans le génome. En effet, l'identification d'une variation du nombre de copies associée par exemple à plus de 250 régions génomiques mais à moins de 5000 régions génomiques réparties de manière aléatoire dans le génome permet d'obtenir un squelette pour l'analyse de la variation du nombre de copies représentatif de la tumeur tout en limitant les coûts de séquençage.

Avantageusement, dans la méthode de caractérisation selon la présente invention, lesdites séquences d'ADN cibles à séquencer contiennent des aberrations génétiques comprenant un phénotype de défaut de recombinaison homologue déterminé par comparaison d'au moins une desdites séquences d'ADN cibles à séquencer par rapport à ladite au moins une séquence d'ADN correspondante dudit génome de référence. Un défaut de recombinaison homologue empêche une réparation normale des dommages de l'ADN, ce qui peut engendrer des aberrations génétiques comme des délétions et/ou des duplications de régions génomiques. Il est donc avantageux de déterminer si la tumeur analysée comprend un phénotype de défaut de recombinaison homologue. En particulier, un phénotype de défaut de recombinaison homologue peut être en partie déterminé par l'état mutationnel des gènes BRCA1 et BRCA2 qui sont impliqués dans un mécanisme de réparation normale des dommages de l'ADN.

De manière préférée, dans la méthode de caractérisation selon la présente invention, lesdites séquences d'ADN cibles à séquencer contiennent des aberrations génétiques comprenant une charge de mutation tumorale déterminée à l'aide d'une comparaison d'une pluralité de séquences codantes desdites séquences d'ADN cibles à séquencer avec une pluralité de séquences codantes correspondantes dudit génome de référence, ladite pluralité de séquences codantes desdites séquences d'ADN cibles à séquencer est déterminée par un séquençage d'au moins 1 mégabase de séquences codantes, préférentiellement au moins 1,1, plus préférentiellement au moins 1,2, 1,3, 1,4, 1,5, 1,6, 1,7, 1,8, 1,9, 2 mégabases de séquences codantes desdites séquences d'ADN cibles à séquencer. En effet, une détermination de la charge de mutation tumorale d'une grande quantité de séquence codante est avantageuse car plus la charge de mutation tumorale est déterminée par une grande quantité de séquence codante, et par un grand panel de gènes, plus la prédiction de la réponse aux thérapies ciblées et à l'immunothérapie sera précise.

De manière préférentielle, dans la méthode de caractérisation d'une tumeur par un séquençage ciblé selon la présente invention, lesdites séquences d'ADN cibles à séquencer contiennent des aberrations génétiques comprenant une instabilité microsatellitaire déterminée à l'aide d'une comparaison d'une série desdites séquences d'ADN cibles à séquencer par rapport à une série de séquences d'ADN correspondante dudit génome de référence, ladite série desdites séquences d'ADN cibles à séquencer est déterminée par un séquençage, d'au moins une séquence d'ADN cibles à séquencer, préférentiellement au moins 100 séquences d'ADN cibles à séquencer, plus préférentiellement au moins 1000 séquences d'ADN cibles à séquencer, de manière favorable au moins 1500 séquences d'ADN cibles à séquencer. En effet, l'instabilité microsatellitaire est un processus impliqué dans le développement tumoral qui peut entraîner un taux de mutation élevé dans la tumeur. La détermination de l'instabilité microsatellitaire peut avoir une valeur prédictive pour déterminer l'efficacité d'une immunothérapie.

Avantageusement, dans la méthode de caractérisation d'une tumeur par un séquençage ciblé selon la présente invention, ladite caractérisation de la tumeur comprend un séquençage d'au moins une partie d'une séquence d'au moins 100, plus particulièrement d'au moins 200, encore plus particulièrement d'au moins 300, de préférence au moins 325, ou mieux, au moins, 350, 375, 400, 425, 450, 475, 500, 525, 550, 575, 600, gènes plus particulièrement de chaque gène d'un panel de gènes listés au Tableau 1 ou d'un séquençage d'au moins une partie d'une séquence de chaque gène d'un sous-groupement de gènes compris dans le Tableau 1 pour identifier une signature d'aberrations génétiques associées à une thérapie. En effet, le séquençage du panel de gènes étendu listé au Tableau 1 permet d'améliorer la détermination d'aberrations génétiques comme l'instabilité microsatellitaire et/ou la charge de mutation tumorale. Les sous-groupements de gènes compris dans le panel de gènes (groupe de gènes) du Tableau 1, dont le séquençage permet d'identifier des aberrations génétiques qui sont associées à des mécanismes associés à une thérapie (traitement), comprennent : (i) un sous-groupement comprenant les gènes EGFR, MET et KRAS pour identifier des traitements basés sur des inhibiteurs de tyrosine kinases de EGFR dans le cadre du cancer du poumon, plus particulièrement du cancer bronchique non à petites cellules (CBNPC), (ii) un sous-groupement comprenant les gènes EGFR, KRAS et BRAF pour identifier des thérapies anti-EGFR dans le cadre du cancer colorectal (CRC), (iii) un sous-groupement comprenant les gènes BRCA1, BRCA2, PALB2 et ATM pour identifier des traitements basés sur des inhibiteurs PARP dans le cadre du cancer du sein ou du cancer de l'ovaire, (iv) un sous-groupement comprenant les gènes APLNR, B2M, PD-L1, PD-L2 pour identifier des traitements basés sur des inhibiteurs anti-PD1 ou anti-PD-L1 dans le cadre de différents cancers comme des cancers du poumon, des mélanomes, ou des cancers de la vessie.

**Tableau 1 : Liste du panel de gènes.**

| | | | | | |
|---|---|---|---|---|---|
| ABL1 | ABL2 | ACVR1 | ACVR1B | AGO1 | AGO2 |
| AJUBA | AKT1 | AKT2 | AKT3 | ALB | ALK |
| ALOX12B | AMER1 | ANKRD11 | ANKRD26 | APC | APLNR |
| AR | ARAF | ARFRP1 | ARHGAP35 | ARID1A | ARID1B |
| ARID2 | ARID5B | ASXL1 | ASXL2 | ATM | ATR |
| ATRX | ATXN7 | AURKA | AURKB | AXIN1 | AXIN2 |
| AXL | B2M | BABAM1 | BAP1 | BARD1 | BAT25 |
| BAT-26 | BBC3 | BCL10 | BCL2 | BCL2L1 | BCL2L11 |
| BCL2L2 | BCL6 | BCOR | BCORL1 | BCR | BIRC3 |
| BLM | BMPR1A | BRAF | BRCA1 | BRCA2 | BRD4 |
| BRIP1 | BTG1 | BTG2 | BTK | C11orf30 | CALR |
| CARD11 | CARM1 | CASP8 | CBFB | CBL | CCNB3 |
| CCND1 | CCND2 | CCND3 | CCNE1 | CD276 | CD70 |
| CD74 | CD79A | CD79B | CDC42 | CDC73 | CDH1 |
| CDK12 | CDK4 | CDK6 | CDK7 | CDK8 | CDKN1A |
| CDKN1B | CDKN2A | CDKN2B | CDKN2C | CEBPA | CENPA |
| CHD2 | CHD4 | CHEK1 | CHEK2 | CIC | CMTR2 |
| CREBBP | CRKL | CRLF2 | CSDE1 | CSF1R | CSF3R |
| CSNK1A1 | CTCF | CTLA4 | CTNNA1 | CTNNB1 | CTR9 |
| CUL3 | CUL4A | CUX1 | CXCR4 | CYLD | CYP17A1 |
| CYP19A1 | CYP2C19 | CYP2D6 | CYSLTR2 | D2S123 | DAXX |
| DCUN1D1 | DDR1 | DDR2 | DDX41 | DHX15 | DICER1 |
| DIS3 | DNAJB1 | DNMT1 | DNMT3A | DNMT3B | DOT1L |
| DPYD | DROSHA | DUSP4 | E2F3 | EED | EGFL7 |
| EGFR | EIF1AX | EIF4A2 | EIF4E | ELF3 | EML4 |
| EMSY | EP300 | EPAS1 | EPCAM | EPHA3 | EPHA5 |
| EPHA7 | EPHB1 | EPHB4 | ERBB2 | ERBB3 | ERBB4 |
| ERCC1 | ERCC2 | ERCC3 | ERCC4 | ERCC5 | ERF |
| ERG | ERRFI1 | ESR1 | ETAA1 | ETS1 | ETV1 |
| ETV4 | ETV5 | ETV6 | EWSR1 | EZH1 | EZH2 |
| EZR | FAM175A | FAM46C | FAM58A | FANCA | FANCC |
| FANCD2 | FANCE | FANCF | FANCG | FANCI | FANCL |
| FAS | FAT1 | FBXW7 | FGF1 | FGF10 | FGF12 |
| FGF14 | FGF19 | FGF2 | FGF23 | FGF3 | FGF4 |
| FGF5 | FGF6 | FGF7 | FGF8 | FGF9 | FGFR1 |
| FGFR2 | FGFR3 | FGFR4 | FH | FLCN | FLI1 |
| FLT1 | FLT3 | FLT4 | FOXA1 | FOXF1 | FOXL2 |
| FOXO1 | FOXP1 | FRS2 | FUBP1 | FYN | GAB1 |
| GAB2 | GABRA6 | GATA1 | GATA2 | GATA3 | GATA4 |
| GATA6 | GEN1 | GID4 | GLI1 | GNA11 | GNA13 |
| GNAQ | GNAS | GNB1 | GPR124 | GPS2 | GREM1 |
| GRIN2A | GRM3 | GSK3B | H3F3A | H3F3B | H3F3C |
| HDAC1 | HGF | HIST1H1C | HIST1H2BD | HIST1H3A | HIST1H3B |
| HIST1H3C | HIST1H3D | HIST1H3E | HIST1H3F | HIST1H3G | HIST1H3H |
| HIST1H3I | HIST1H3J | HIST2H3A | HIST2H3C | HIST2H3D | HIST3H3 |
| HLA-A | HLA-B | HLA-C | HNF1A | HNRNPK | HOXB13 |
| HRAS | HSD3B1 | HSP90AA1 | ICOSLG | ID3 | IDH1 |
| IDH2 | IFNGR1 | IGF1 | IGF1R | IGF2 | IKBKE |
| IKZF1 | IL10 | IL7R | INHA | INHBA | INPP4A |
| INPP4B | INPPL1 | INSR | IRF2 | IRF4 | IRS1 |
| IRS2 | JAK1 | JAK2 | JAK3 | JUN | KAT6A |
| KBTBD4 | KDM5A | KDM5C | KDM6A | KDR | KEAP1 |
| KEL | KIF5B | KIT | KLF4 | KLF5 | KLHL6 |
| KMT2A | KMT2B | KMT2C | KMT2D | KMT5A | KNSTRN |
| KRAS | LAMP1 | LATS1 | LATS2 | LMO1 | LRP1B |
| LTK | LYN | LZTR1 | MAD2L2 | MAGI2 | MALT1 |
| MAP2K1 | MAP2K2 | MAP2K4 | MAP3K1 | MAP3K13 | MAP3K14 |
| MAP3K4 | MAPK1 | MAPK3 | MAPKAP1 | MAX | MCL1 |
| MDC1 | MDM2 | MDM4 | MED12 | MEF2B | MEN1 |
| MET | MGA | MITF | MLH1 | MLLT1 | MLLT3 |
| MPL | MRE11A | MSH2 | MSH3 | MSH6 | MSI1 |
| MSI2 | MST1 | MST1R | MTAP | MTOR | MUTYH |
| MYB | MYC | MYCL | MYCN | MYD88 | MYOD1 |
| NAB2 | NADK | NBN | NCOA3 | NCOR1 | NEGR1 |
| NF1 | NF2 | NFE2L2 | NFKBIA | NKX2-1 | NKX3-1 |
| NOTCH1 | NOTCH2 | NOTCH3 | NOTCH4 | NPM1 | NR-21 |
| NR-27 | NRAS | NRG1 | NSD1 | NT5C2 | NTHL1 |
| NTRK1 | NTRK2 | NTRK3 | NUF2 | NUP93 | NUTM1 |
| P2RY8 | PAK1 | PAK3 | PAK7 | PALB2 | PARK2 |
| PARP1 | PARP2 | PARP3 | PAX3 | PAX5 | PAX7 |
| PAX8 | PBRM1 | PD-1 | PDGFRA | PDGFRB | PDK1 |
| PD-L1 | PD-L2 | PDPK1 | PGBD5 | PGR | PHF6 |
| PHOX2B | PIGA | PIK3C2B | PIK3C2G | PIK3C3 | PIK3CA |
| PIK3CB | PIK3CD | PIK3CG | PIK3R1 | PIK3R2 | PIK3R3 |
| PIM1 | PLCG2 | PLK2 | PMAIP1 | PMS1 | PMS2 |
| PNRC1 | POLD1 | POLE | POT1 | PPARG | PPM1D |
| PPP2R1A | PPP2R2A | PPP4R2 | PPP6C | PRDM1 | PRDM14 |
| PREX2 | PRKAR1A | PRKCI | PRKD1 | PRKDC | PRSS8 |
| PTCH1 | PTEN | PTP4A1 | PTPN11 | PTPRD | PTPRS |
| PTPRT | QKI | RAB35 | RAC1 | RAC2 | RAD21 |
| RAD50 | RAD51 | RAD51B | RAD51C | RAD51D | RAD52 |
| RAD54L | RAF1 | RANBP2 | RARA | RASA1 | RB1 |
| RBM10 | RECQL | RECQL4 | REL | REST | RET |
| RFWD2 | RHEB | RHOA | RICTOR | RIT1 | RNF43 |
| ROS1 | RPS6KA4 | RPS6KB1 | RPS6KB2 | RPTOR | RRAGC |
| RRAS | RRAS2 | RSPO2 | RTEL1 | RUNX1 | RUNX1T1 |
| RXRA | RYBP | SCG5 | SDC4 | SDHA | SDHAF2 |
| SDHB | SDHC | SDHD | SERPINB3 | SERPINB4 | SESN1 |
| SESN2 | SESN3 | SETBP1 | SETD2 | SETDB1 | SF3B1 |
| SGK1 | SH2B3 | SH2D1A | SHOC2 | SHQ1 | SLC34A2 |
| SLFN11 | SLIT2 | SLX4 | SMAD2 | SMAD3 | SMAD4 |
| SMARCA2 | SMARCA4 | SMARCB1 | SMARCD1 | SMARCE1 | SMC1A |
| SMC3 | SMO | SMYD3 | SNCAIP | SOCS1 | SOS1 |
| SOX10 | SOX17 | SOX2 | SOX9 | SPEN | SPOP |
| SPRED1 | SPRTN | SPTA1 | SRC | SRSF2 | STAG1 |
| STAG2 | STAT3 | STAT4 | STAT5A | STAT5B | STK11 |
| STK19 | STK40 | SUFU | SUZ12 | SYK | TAF1 |
| TAP1 | TAP2 | TBX3 | TCEB1 | TCF3 | TCF7L2 |
| TEK | TERT | TET1 | TET2 | TFE3 | TFRC |
| TGFBR1 | TGFBR2 | TIPARP | TMEM127 | TMPRSS2 | TNFAIP3 |
| TNFRSF14 | TOP1 | TOP2A | TP53 | TP53BP1 | TP63 |
| TPMP | TRAF2 | TRAF7 | TRIP13 | TSC1 | TSC2 |
| TSHR | TYRO3 | U2AF1 | UGT1A1 | UPF1 | USP8 |
| VEGFA | VHL | VTCN1 | WHSC1 | WHSC1L1 | WISP3 |
| WT1 | WWTR1 | XIAP | XPO1 | XRCC2 | YAP1 |
| YES1 | ZBTB2 | ZBTB7A | ZFHX3 | ZNF217 | ZNF703 |
| ZNRF3 | ZRSR2 | | | | |

Préférentiellement, ladite caractérisation de la tumeur comprend une identification d'une aberration génétique par rapport audit génome de référence d'au moins une séquence codante ou non-codante d'un gène associé à un traitement contre le cancer, et/ou d'au moins une séquence non-codante au sein d'une séquence d'un gène associée aux translocations associées à un traitement contre le cancer et/ou d'au moins une région d'épissage d'au moins un gène associée à un traitement contre le cancer. En effet, cette caractérisation de la tumeur permet de mieux cibler le traitement contre le cancer adapté aux aberrations génétiques spécifiques de la tumeur.

Préférentiellement, lesdites séquences d'ADN cibles à séquencer contiennent des aberrations génétiques comprenant un déséquilibre allélique des télomères par rapport audit génome de référence identifié par au moins deux, préférentiellement au moins 100, plus préférentiellement au moins 1000, de manière favorable au moins 2000, et de préférence maximum 20000, plus préférentiellement maximum 10000, de manière favorable maximum 6000 polymorphismes d'un seul nucléotide et/ou insertions et/ou délétions d'un nucléotide d'au moins 1 fragment d'ADN localisé dans une région pré-télomérique. Avantageusement, les polymorphismes d'un seul nucléotide sont déterminés avec une fréquence des allèles mineurs égale ou supérieure à 20%, préférentiellement égale ou supérieure à 25%, de manière favorable égale ou supérieure à 30%. En effet, un déséquilibre allélique des télomères est indicatif d'un mécanisme de réparation de l'ADN défectueux et prédit une sensibilité accrue de la tumeur à des agents d'altération de l'ADN comme des agents à base de platine. Selon la présente invention, une région pré-télomérique est définie par exemple par son contenu ayant des séquences répétitives de l'ADN et par le fait qu'elle ne contient pas de gènes.

De manière préférée, lesdites séquences d'ADN cibles à séquencer contiennent des aberrations génétiques comprenant un phénotype de défaut de recombinaison homologue HRD, ledit phénotype HRD étant déterminé par (i) une identification d'une série de polymorphisme d'un seul nucléotide d'au moins une séquence d'ADN cible à séquencer par rapport à au moins une séquence d'ADN correspondante dudit génome de référence, ladite au moins une séquence d'ADN cible à séquencer étant localisée dans au moins une région génomique au sein d'un gène et, (ii) une identification d'une série de polymorphisme d'un seul nucléotide et/ou d'une série d'insertion et/ou d'une série de délétion d'un nucléotide par comparaison d'au moins une séquence d'ADN cible à séquencer localisée dans une région pré-télomérique par rapport à au moins une séquence d'ADN correspondante localisée dans une région pré-télomérique correspondante dudit génome de référence, ladite série de polymorphisme d'un seul nucléotide d'au moins une séquence d'ADN cible à séquencer localisée dans une région pré-télomérique est déterminée avec une fréquence des allèles mineurs égale ou supérieure à 20%, préférentiellement égale ou supérieure à 25%, de manière favorable égale ou supérieure à 30%. En effet, une analyse de perte d'hétérozygotie à l'aide d'une identification combinée d'un polymorphisme d'un seul nucléotide au sein d'un gène et d'un polymorphisme d'un seul nucléotide au sein d'une région pré-télomérique déterminé avec une fréquence des allèles mineurs suffisante permet d'établir un phénotype HRD tout en réduisant le pourcentage de faux négatif pour ce phénotype.

De manière avantageuse, lesdites séquences d'ADN cibles à séquencer contiennent des aberrations génétiques comprenant au moins 2, préférentiellement au moins 3, plus préférentiellement au moins 4, encore plus préférentiellement au moins 5, de manière favorable au moins 6 aberrations génétiques choisies dans un groupe d'aberrations génétiques comprenant un polymorphisme d'un seul nucléotide, une délétion d'une copie ou de deux copies d'un gène, une variation du nombre de copies, un phénotype de défaut de recombinaison homologue, une charge de mutation tumorale, une instabilité microsatellitaire, un déséquilibre allélique des télomères, ou une mutation, une translocation ou un épissage associé à un traitement contre le cancer, lesdites aberrations génétiques de la tumeur étant déterminées par comparaison desdites séquences d'ADN cibles à séquencer par rapport aux séquences d'ADN correspondantes du génome de référence. Il est en effet avantageux de déterminer un ensemble d'aberrations génétiques de manière à obtenir une caractérisation précise et complète de la tumeur. L'identification d'une combinaison d'aberrations génétiques permet en outre de mieux définir la sensibilité de la tumeur à des traitements contre le cancer.

Avantageusement, ladite caractérisation de la tumeur comprend en outre une identification d'une expression d'au moins un marqueur tumoral mesurée par son taux d'ARN et/ou de microARN et/ou de protéine. Avantageusement, la mesure de l'expression d'au moins un marqueur tumoral, au niveau ARN et/ou microARN et/ou protéique, étant une mesure par comparaison de l'expression dudit au moins un marqueur tumoral par rapport à l'expression dudit au moins un marqueur dans le tissu sain correspondant provenant du patient. En effet, la caractérisation de la tumeur sera d'autant plus complète si elle incorpore, en plus d'une caractérisation des aberrations génétiques, une mesure d'expression de marqueurs ARN, microARN et/ou protéique de la tumeur. Ces mesures d'expression enrichissent la caractérisation génétique de la tumeur pour fournir une plus vue globale, plus fonctionnelle des mécanismes moléculaires en cours dans la tumeur.

Avantageusement, dans la méthode de caractérisation selon la présente invention, ladite pluralité de sondes d'hybridation d'ADN dénaturées spécifiques à ladite pluralité desdits fragments d'ADN bloqués est un mélange contenant plusieurs sondes d'hybridation de séquences identiques ou différentes mais complémentaires à au moins 60%, préférentiellement au moins 70%, plus préférentiellement au moins 80%, encore plus préférentiellement au moins 90%, de manière préférée au moins 95%, 96%, 97%, 98%, 99% de la séquence d'un ou de plusieurs fragments de la pluralité desdits fragments d'ADN bloqués, et où ladite pluralité de sondes d'hybridation d'ADN dénaturées spécifiques à ladite pluralité desdits fragments d'ADN bloqués est une sonde d'ADN dénaturée par fragment d'ADN bloqué ou plusieurs sondes d'ADN dénaturée par fragment d'ADN bloqué. En effet, lorsque le fragment d'ADN bloqué à enrichir est un fragment avec une séquence complexe, l'hybridation de plus d'une sonde d'ADN dénaturée par fragment d'ADN bloqué peut améliorer l'efficacité d'enrichissement, ce qui augmente l'uniformité d'enrichissement que ce soit pour des fragments d'ADN contenant des séquences simples ou des fragments d'ADN contenant des séquences complexes.

De manière avantageuse, lesdites séquences d'ADN cibles à séquencer possèdent une séquence d'une longueur moyenne comprise entre 10 et 10000 paires de bases, préférentiellement entre 100 et 1000 paires de bases, plus préférentiellement entre 200 et 600 paires de bases, de manière favorable entre 375 et 425 paires de bases. En effet, l'obtention d'un ensemble de séquences d'ADN cibles à séquencer ayant une longueur de séquence homogène est un critère qui permet d'augmenter l'uniformité de séquençage.

Avantageusement, la méthode de caractérisation selon la présente invention identifie un traitement basé sur la caractérisation de la tumeur. En effet, le traitement ainsi identifié par la caractérisation de la tumeur est spécifique des aberrations génétiques de la tumeur.

Préférentiellement, la méthode de caractérisation selon la présente invention comprend en outre un établissement d'une cartographie de la tumeur dans laquelle les aberrations génétiques de la tumeur sont indiquées en regard de traitements recommandés pour les tumeurs caractérisées par ces aberrations génétiques, lesdits traitements recommandés étant obtenus par comparaison des aberrations génétiques de la tumeur analysée avec des aberrations génétiques de tumeurs de référence et de leurs traitements thérapeutiques de référence enregistrés dans une base de données.

D'autres modes de réalisation de la méthode selon la présente invention sont indiquées dans les revendications annexées.

La présente invention se rapporte aussi à un agent ou plusieurs agents pour traiter la tumeur d'un patient dont ladite tumeur a été caractérisée par la méthode selon la présente invention, ledit agent ou plusieurs agents étant choisi dans le groupe comprenant des inhibiteurs PARP, des thérapies ciblées, des immunothérapies, des chimiothérapies, des radiothérapies, des agents endommageant l'ADN comme des agents à base de platine, des thérapies adjuvantes, des inhibiteurs de tyrosine kinases, des inhibiteurs du point de contrôle immunitaire, l'erlotinib, le gefitinib, l'afatinib, l'osimertinib, le dacomitinib, le cabozantinib, le crizotinib, l'alectinib, le ceritinib, le brigatinib, le vemurafenib, l'encorafenib, le dabrafenib, le trametinib, le cobimetinib, le docetaxel, le paclitaxel, le gemcitabine, le pemetrexed, le pembrolizumab, l'atezolizumab, le nivolumab, le durvalumab, l'olaparib, le niraparib, le talazoparib, le rucaparib, le trastuzumab, le pertuzumab, le neratinib, un traitement ou une thérapie approuvée ou en cours de développement.

D'autres modes de réalisation d'un agent ou d'une combinaison d'agents pour traiter la tumeur d'un patient selon la présente invention sont indiquées dans les revendications annexées.

La présente invention se rapporte aussi à l'utilisation d'une pluralité de sondes d'hybridation d'ADN double brin pour réaliser un séquençage ciblé de fragments d'ADN contenant des séquences simples et de fragments d'ADN contenant des séquences complexes d'un échantillon d'un patient atteint d'un cancer, ledit échantillon comprenant au moins une cellule tumorale.

D'autres modes de réalisation de l'utilisation d'une pluralité de sondes d'hybridation d'ADN double brin selon la présente invention sont indiquées dans les revendications annexées.

La présente invention se rapporte aussi à une méthode pour le traitement du cancer chez un patient comprenant les étapes de a) une caractérisation de la tumeur du patient, b) une détermination du traitement ou des traitements à administrer comprenant un ou plusieurs agents choisi dans le groupe comprenant des inhibiteurs PARP, des thérapies ciblées, des immunothérapies, des chimiothérapies, des radiothérapies, des agents endommageant l'ADN comme des agents à base de platine, des thérapies adjuvantes, des inhibiteurs de tyrosine kinases, des inhibiteurs du point de contrôle immunitaire, l'erlotinib, le gefitinib, l'afatinib, l'osimertinib, le dacomitinib, le cabozantinib, le crizotinib, l'alectinib, le ceritinib, le brigatinib, le vemurafenib, l'encorafenib, le dabrafenib, le trametinib, le cobimetinib, le docetaxel, le paclitaxel, le gemcitabine, le pemetrexed, le pembrolizumab, l'atezolizumab, le nivolumab, le durvalumab, l'olaparib, le niraparib, le talazoparib, le rucaparib, le trastuzumab, le pertuzumab, le neratinib, un traitement ou une thérapie approuvée ou en cours de développement, et c) une administration du traitement ou des traitements au patient, caractérisée en ce que la caractérisation de la tumeur est mise en œuvre en appliquant la méthode de caractérisation de la tumeur selon la présente invention.

Avantageusement, la méthode pour le traitement du cancer chez un patient selon la présente invention comprend l'étape de répéter au cours du temps la caractérisation de la tumeur du patient afin de déterminer si un autre traitement doit être administré audit patient.

D'autres modes de réalisation de la méthode pour le traitement du cancer selon la présente invention sont indiquées dans les revendications annexées.

La présente invention se rapporte enfin à un rapport théranostique d'un patient atteint d'un cancer, obtenu en mettant en œuvre la méthode de caractérisation de la tumeur selon la présente invention lorsqu'elle comprend l'étape d'établissement de la cartographie susdit, destiné à être utilisé pour déterminer un traitement ou une thérapie approuvée ou en cours de développement du cancer du patient.

D'autres caractéristiques, détails et avantages de l'invention ressortiront de la description donnée ci-après, à titre non limitatif et en faisant référence aux dessins annexés.
La figure 1 est une représentation schématique des différentes étapes de la méthode de caractérisation d'une tumeur par un séquençage ciblé à partir d'un échantillon d'un patient atteint d'un cancer.
La figure 2 est un schéma d'une sonde d'hybridation d'ADN double brin (sonde ADN double brin).
La figure 3 est un schéma expliquant l'utilisation d'une pluralité de sondes d'hybridation d'ADN double brin en oncologie pour réaliser un séquençage ciblé d'une tumeur.
La figure 4 montre un schéma reprenant les étapes d'utilisation de la méthode de caractérisation d'une tumeur afin d'identifier un traitement adapté et d'établir un rapport théranostique de la tumeur.

Sur les figures, les éléments identiques ou analogues portent les mêmes références. Les flèches noires indiquent le sens de progression à suivre.

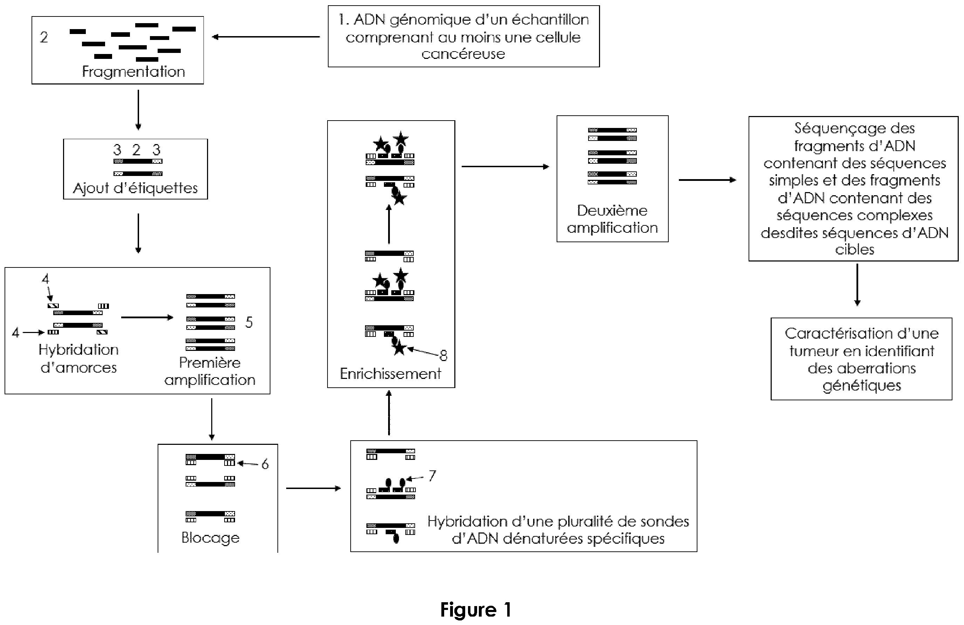

La figure 1 illustre un exemple des différentes étapes de la méthode de caractérisation d'une tumeur par un séquençage ciblé à partir d'un échantillon d'un patient atteint d'un cancer. Chaque cadre de la figure 1 représente une étape dans une succession d'étapes de la méthode de caractérisation de la tumeur. Dans une première étape 1, l'ADN génomique d'un échantillon comprenant au moins une cellule cancéreuse, ADN génomique de la tumeur, est récolté. Préférentiellement, l'échantillon peut être n'importe quel échantillon d'une tumeur solide comme des tissus fixés au formol et inclus dans la paraffine (FFPE) ou des tissus congelés. Préférentiellement, la quantité initiale d'ADN génomique utilisé pour la méthode est égale ou supérieure à 10 ng, plus préférentiellement d'environ 50 ng. Cet ADN génomique est ensuite fragmenté, lors d'une étape de fragmentation, pour donner des fragments d'ADN 2. Cette étape de fragmentation peut être réalisée par une fragmentation physique, mécanique, comme par exemple une sonication, ou une fragmentation enzymatique comme par exemple l'utilisation d'endonucléase, ou une fragmentation chimique. La durée de l'étape de fragmentation peut être comprise entre 1min et 1h, préférentiellement entre 5min et 25min. Des étiquettes sont ensuite ajoutées à ces fragments d'ADN 2. Pour cela, ces fragments d'ADN 2 subissent une étape de réparation des bouts de séquence de manière à ne pas avoir de nucléotide non appariés 'end repair' (étape non représentée sur la figure 1). Ces fragments d'ADN 2 sont ensuite polyadénylés (étape non représentée sur la figure 1) et vont subir une étape de ligation d'adaptateurs universels. Les étapes de réparation des bouts des fragments d'ADN, de polyadénylation et de ligation des adaptateurs universels sont schématisées de manière globale par l'étape d'ajout d'étiquettes 3 représenté à la figure 1. Les étiquettes comprenant des adaptateurs universels permettent entre autres une hybridation d'amorces. Ces fragments d'ADN, portant les étiquettes ajoutées 3, peuvent ensuite être sélectionnés en fonction de leur taille (étape non représentée sur la figure 1). Préférentiellement, les fragments d'ADN à séquencer possèdent une séquence d'une longueur moyenne comprise entre 10 et 10000 paires de bases, préférentiellement entre 100 et 1000 paires de bases, plus préférentiellement entre 200 et 600 paires de bases, de manière favorable entre 375 et 425 paires de bases. Des fragments d'ADN ayant une longueur de séquence similaires facilitent l'uniformité du séquençage ciblé. Les fragments d'ADN récoltés sont ensuite amplifiés lors d'une étape de première amplification 5. Cette étape de première amplification peut être une amplification PCR. Pour ce faire, des amorces PCR 4 ayant une séquence complémentaire à au moins une partie d'une séquence des étiquettes vont s'hybrider aux étiquettes et initier la première amplification 5. Les étapes de fragmentation 2, d'ajout d'étiquettes 3, d'hybridation d'amorce PCR 4 et de première amplification 5 permettent la préparation d'une bibliothèque de séquençage et donne un premier mélange comprenant des fragments d'ADN amplifiés.

Les fragments d'ADN amplifiés par la première amplification 5 sont ensuite bloqués, lors d'une étape de blocage, à l'aide de bloqueurs universels 6. Le blocage des fragments d'ADN amplifiés se réalise à l'aide de bloqueurs universels 6 ayant une séquence complémentaire à au moins une partie de la séquence des étiquettes. Ces bloqueurs universels 6 empêchent l'hybridation non-spécifique entre les séquences des étiquettes et permettent de la sorte un meilleur enrichissement subséquent des fragments d'ADN cibles. De plus, le génome comprend énormément de séquences d'ADN répétitives dont on veut se débarrasser avant le séquençage. Lors de l'étape de blocage, un ajout d'ADN Cot-1 en plus des bloqueurs universels 6 permet de se débarrasser des séquences répétitives d'ADN présentes dans le génome (étape non représentée à la figure 1). En effet, l'ADN Cot-1 est un échantillon contenant des séquences d'ADN complémentaires à la plupart des séquences d'ADN répétitives du génome humain et pratiquement aucune des séquences uniques (non répétitives) du génome humain. L'ADN Cot-1 permet donc par une hybridation par complémentarité de se débarrasser d'une grande partie des séquences répétitives du génome. La longueur moyenne des fragments d'ADN dans un tel échantillon est d'environ 300 paires de bases (pb). L'ADN Cot-1 permet de supprimer les séquences répétitives d'ADN dans le cadre de l'hybridation génomique.

La figure 1 illustre ensuite l'hybridation d'une pluralité desdits fragments d'ADN bloqués par ladite pluralité de sondes d'hybridation d'ADN dénaturées spécifiques à ladite pluralité desdits fragments d'ADN bloqués pour former un deuxième mélange comprenant des fragments d'ADN hybridés et des fragments d'ADN non-hybridés à la pluralité de sondes d'hybridation d'ADN double brin (point 7 à la figure 1). La pluralité de sondes d'hybridation d'ADN dénaturées spécifiques provenant de la dénaturation de la pluralité de sondes d'hybridation d'ADN double brin spécifiques. En effet, préalablement à l'étape d'hybridation entre lesdits fragments d'ADN bloqués et ladite pluralité de sondes d'hybridation d'ADN dénaturées spécifiques, les sondes d'hybridation d'ADN double brin sont dénaturées, de préférence par chauffage à une température avantageusement comprise entre 95°C et 99 °C, pour former des sondes d'hybridation d'ADN dénaturées. Dans le contexte de la présente invention, une hybridation entre une sonde d'hybridation ADN double brin et un fragment d'ADN provenant de l'échantillon du patient signifie une hybridation entre une sonde d'ADN double brin préalablement dénaturée en deux sondes d'ADN simple brin où chaque sonde d'ADN simple brin formant préalablement la sonde d'ADN double brin s'hybride avec un brin du fragment d'ADN, séquence d'ADN cible, provenant de l'échantillon du patient. Dans un mode de réalisation particulier de la méthode selon la présente invention, cette étape d'hybridation d'une pluralité desdits fragments d'ADN bloqués par une pluralité de sondes d'hybridation d'ADN dénaturées spécifiques à ladite pluralité desdits fragments d'ADN bloqués est réalisée à une température comprise entre 40 et 90°C, plus préférentiellement entre 60 et 80°C, de manière favorable à 70°C pendant une période de temps comprise entre 1h et 40h, préférentiellement entre 10 et 20h, de manière favorable de 16h.

Une étape d'enrichissement (point 8 de la figure 1) est réalisée par un enrichissement uniforme et simultané de fragments d'ADN contenant des séquences simples et des fragments d'ADN contenant des séquences complexes desdits fragments d'ADN hybridés à la pluralité de sondes d'hybridation d'ADN dénaturées dudit deuxième mélange par une capture desdits fragments d'ADN hybridés pour former un milieu enrichi en lesdits fragments d'ADN hybridés à la pluralité de sondes d'hybridation d'ADN dénaturées. De manière favorable, les séquences complexes sont définies par un pourcentage de base nucléotidique guanine et cytosine égal ou supérieur à 80%. De manière favorable, l'étape de blocage (point 6 de la figure 1) et l'étape d'hybridation des fragments d'ADN bloqués réalisée à l'aide d'une pluralité de sondes d'hybridation d'ADN dénaturées spécifiques (point 7 de la figure 1) se réalisent de manière concomitante. De manière avantageuse, la capture des fragments d'ADN hybridés est effectuée à l'aide de billes de streptavidine magnétiques (point 8 à la figure 1). Ces billes de streptavidine sont des particules superparamagnétique couplées de manière covalente à des protéines de streptavidine. La capture des fragments d'ADN hybridés est permise à l'aide d'une application d'un champ magnétique et à une très haute affinité existant entre une protéine de streptavidine et une molécule de biotine qui permet de conserver uniquement les fragments d'ADN hybridés aux sondes d'hybridation d'ADN dénaturées. De manière préférée, le rapport entre la quantité de billes de streptavidine et la quantité de sondes d'hybridation d'ADN double brin est comprise entre 0,5 et 3, plus préférentiellement entre 1 et 2, de manière favorable de 1,4. Le temps d'incubation des fragments d'ADN hybridés aux couples sondes ADN dénaturées - biotine avec les billes de streptavidine est compris entre 1 min et 5 h, préférentiellement entre 10 min et 1 h, de manière favorable de 30 min.

Après l'étape d'enrichissement, une étape de lavage et une récupération desdits fragments d'ADN hybridés à la pluralité de sondes d'hybridation d'ADN dénaturées permettent de former un milieu enrichi avec des séquences d'ADN cibles. Cette étape de lavage et de récupération est effectuée entre les étapes d'enrichissement d'un échantillon d'ADN à séquencer et de la deuxième amplification. En particulier, une ou plusieurs étapes de lavage suivie par une étape de récupération peuvent être réalisées (étapes non représentées à la figure 1). Ces étapes assurent une purification optimale des fragments d'ADN à séquencer. Préférentiellement, une première étape de lavage est réalisée avec un premier tampon de lavage ayant une température comprise entre 20 et 30°C, de manière favorable de 25°C, suivie par une deuxième étape de lavage avec un deuxième tampon de lavage ayant une température comprise entre 40 et 60°C, de manière favorable de 48°C.

Après les étapes de lavage et de récupération qui permettent de se débarrasser des bloqueurs et de ce qui n'est pas hybridés à la pluralité de sondes d'hybridation d'ADN dénaturées (étapes non représentées à la figure 1), une deuxième amplification, amplification PCR post-capture, est réalisée (voir figure 1). Cette deuxième amplification desdites séquences d'ADN cibles se réalise à l'aide d'amorces ayant une séquence complémentaire à une séquence desdites étiquettes donnant des séquences d'ADN cibles à séquencer. Cette deuxième amplification permet d'obtenir suffisamment de séquences d'ADN cibles à séquencer pour le séquençage subséquent. De manière préférentielle, cette deuxième amplification compte entre 5 et 15 cycles PCR, de manière favorable 9 cycles PCR.

De manière favorable, les différentes étapes décrites ci-dessus permettent d'obtenir un échantillon comprenant des séquences d'ADN cibles à séquencer d'une grande qualité définie par une concentration des séquences d'ADN cibles à séquencer égale ou supérieure à 15 ng/µl et par des séquences d'ADN cibles à séquencer possédant une séquence définie par une longueur moyenne comprise entre 375 et 425 paires de bases.

Après la deuxième amplification, un séquençage ciblé des séquences d'ADN cibles à séquencer est réalisé (voir figure 1). Ce séquençage ciblé, qui génère des données de séquençage, est un séquençage simultané des fragments d'ADN contenant des séquences simples et des fragments d'ADN contenant des séquences complexes dans les mêmes conditions de séquençage. De manière avantageuse, le séquençage est réalisé par un séquenceur NextSeq500/550 ou n'importe quel séquenceur Illumina ou MGI. Préférentiellement, le séquençage est un séquençage à extrémités jumelés *'paired* end sequencing', mais peut également être un séquençage à extrémité unique 'single end sequencing'.

A l'aide d'algorithmes d'analyse bioinformatique, comme par exemple ABRA (https://academic.oup.com/bioinformatics/orticle/30/19/2813/2422200), SAMTOOLS (https://www.ncbi.nlm.nih.aov/pmc/articles/PMC2723002/), BEDTOOLS (https://academic.oup.com/bioinformatics/orticle/26/6/841/244688), ou FASTP (https://academic.oup.com/bioinformatics/orticle/34/17/i884/5093234), les données obtenues par le séquençage ciblé ont permis une caractérisation de la tumeur par un séquençage des fragments d'ADN contenant des séquences simples et des fragments d'ADN contenant des séquences complexes desdites séquences d'ADN cibles à séquencer de manière à identifier des aberrations génétiques de la tumeur (voir dernière étape de la figure 1).

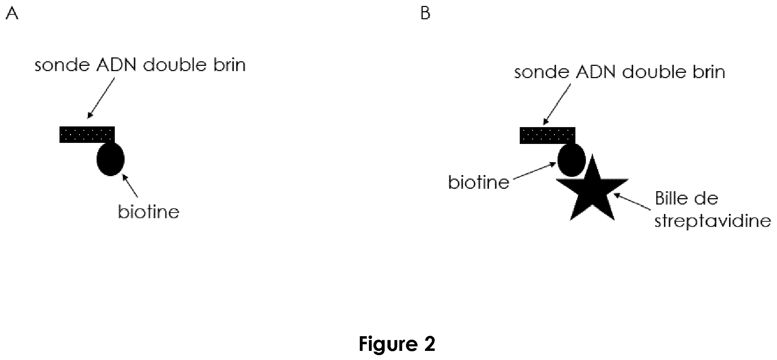

La figure 2 illustre une représentation schématique d'une sonde d'hybridation d'ADN double brin (sonde ADN double brin) couplée à une molécule de biotine (point A de la figure 2). Le point B de la figure 2 illustre l'affinité entre la molécule de biotine et la protéine streptavidine qui est liée de manière covalente à une particule superparamagnétique pour former la bille de streptavidine. Le fragment d'ADN hybridé à la sonde d'hybridation d'ADN double brin qui sera enrichi pour être séquencé n'est pas représenté à la figure 2.

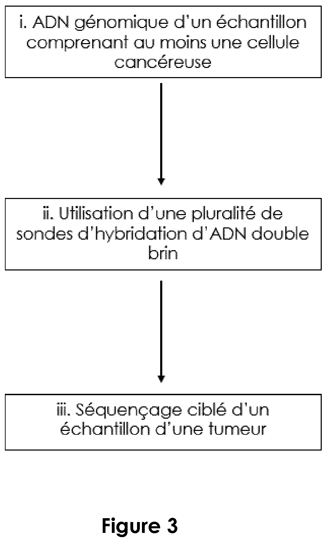

La figure 3 illustre l'utilisation d'une pluralité de sondes d'hybridation d'ADN double brin pour réaliser un séquençage ciblé de fragments d'ADN contenant des séquences simples et de fragments d'ADN contenant des séquences complexes d'un échantillon d'un patient atteint d'un cancer, l'échantillon comprenant au moins une cellule tumorale. Chaque cadre de la figure 3 représente une étape pour l'utilisation d'une pluralité de sondes d'hybridation d'ADN double brin en oncologie. En effet, l'utilisation d'une pluralité de sondes d'hybridation d'ADN double brin ii à partir d'ADN génomique d'un échantillon comprenant au moins une cellule cancéreuse i permet de réaliser un séquençage ciblé de la tumeur iii. L'utilisation d'une pluralité de sondes d'hybridation d'ADN double brin sur un échantillon ADN provenant d'une tumeur permet notamment un enrichissement et un séquençage ciblé de la tumeur uniforme, ce qui limite les coûts de séquençage tout en diminuant les nombre de faux négatif et faux positif récoltés dans les données de séquençage.

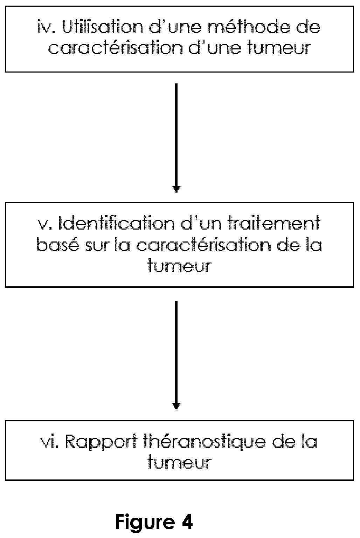

La figure 4 illustre les étapes qui permettent d'utiliser la méthode de caractérisation d'une tumeur iv décrite ci-avant pour identifier un traitement basé sur la caractérisation de la tumeur v. De plus, en utilisant la méthode de caractérisation de la tumeur décrite ci-avant, qui permet d'identifier des aberrations génétiques de la tumeur et de prédire un traitement adapté basé sur la caractérisation de la tumeur, un rapport théranostique de la tumeur peut être généré vi. Chaque cadre de la figure 4 représente une étape dans l'utilisation de la méthode de caractérisation pour identifier un traitement adapté et pour établir un rapport théranostique de la tumeur.

Avantageusement, le rapport théranostique de la tumeur peut comprendre des informations médicales et/ou des données du séquençage ciblé de la tumeur avec une liste des aberrations génétiques identifiées et/ou des données de caractérisations additionnelles de la tumeur comme des mesures d'expression de marqueurs tumoraux au niveau ARN et/ou microARN et/ou protéique, et/ou un immunogramme définissant un potentiel de réponse à une immunothérapie basé sur la caractérisation de la tumeur, et/ou une liste de traitements avec leurs propriétés qui pourraient être associés à un bénéfice clinique et/ou ceux qui ne seraient pas associés à un bénéfice, et/ou une liste des essais cliniques associés à la caractérisation de la tumeur et/ou une liste des publications en lien avec la caractérisation de la tumeur.

### Exemples.-

### Exemple 1.- Récolte d'ADN génomique à partir d'un échantillon d'une tumeur solide et préparation de cet ADN pour le séquençage

Dans une première étape de la méthode de caractérisation d'une tumeur selon la présente invention, de l'ADN génomique est récolté à partir d'un échantillon comprenant au moins une cellule cancéreuse. Pour ce faire, les étapes suivantes sont mises en place :
- une réception d'un échantillon formé d'un bloc comprenant un morceau de tumeur extrait par une biopsie fixé au formol et inclus dans la paraffine (bloc FFPE) ;
- un découpage du bloc en lames d'une épaisseur de 7 µm;
- une coloration de la première et dernière lame avec de l'hematoxiline éosine (lames H&E) ;
- une identification à partir de la première lame, par inspection visuelle, d'une zone de la lame comprenant suffisamment de cellules tumorales, peu ou pas de nécrose et une infiltration lymphocytaire inférieure à 21%;
- sur cette première lame colorée, un marquage d'une région comprenant le plus de cellules tumorales;
- un report du marquage de la première lame sur les lames non colorée pour marquer la même région de la tumeur, mais sur une tranche différente ;
- un grattage des cellules de ces régions marquées (étape de macrodissection) ;
- une extraction de l'ADN de ces lames pour former l'ADN génomique à caractériser ;
- en parallèle, une quantification de l'ADN à l'aide d'un spectrophotomètre à partir d'un échantillon de l'ADN génomique à caractériser;
- en parallèle, une qualification de l'ADN par migration sur gel à partir d'un échantillon de l'ADN génomique à caractériser.

L'échantillon d'ADN génomique peut ensuite être caractérisé en appliquant le protocole de la figure 1.

### Exemple 2.- Enrichissement de fragments d'ADN contenant des séquences simples et de fragments d'ADN contenant des séquences complexes desdits fragments d'ADN hybridés à la pluralité de sondes d'hybridation d'ADN dénaturées

Pour enrichir des fragments d'ADN contenant des séquences simples et des fragments d'ADN contenant des séquences complexes desdits fragments d'ADN hybridés à la pluralité de sondes d'hybridation d'ADN dénaturées, le protocole de la figure 1 a été appliqué sur l'échantillon tumoral de l'exemple 1. Plus précisément, on a fragmenté 50 ng d'ADN génomique de l'échantillon par sonication pendant 10 min, ce qui a donné des fragments d'ADN. On a ensuite ajouté des étiquettes aux extrémités des fragments d'ADN selon un protocole standard bien connu de l' homme de métier. On en ensuite amplifié ces fragments d'ADN lors d'une étape de première amplification PCR comprenant 8 cycles d'amplification PCR pour donner des fragments d'ADN amplifiés. Ces fragments d'ADN amplifiés ont ensuite été simultanément bloqués à l'aide de bloqueurs universels et hybridés à l'aide de sondes d'hybridation d'ADN double brin où le temps d'hybridation est de 16h à 70°C pour donner des fragments d'ADN hybridés. Préalablement à l'étape d'hybridation, une étape de dénaturation par chauffage à une température de 98°C pendant 15 secondes a été réalisée pour dénaturer les fragments d'ADN et les sondes d'hybridation d'ADN double brin formant de la sorte un mélange de fragments d'ADN simple brin et de sondes d'hybridation d'ADN dénaturées simple brin.

Par la suite, les fragments d'ADN hybridés ont été capturés à l'aide de billes de streptavidine magnétiques où le temps d'incubation avec les billes est de 30 min avec un rapport de bille par rapport aux sondes d'hybridation d'ADN double brin de 1,4 pour donner un mélange enrichi en des fragments d'ADN hybridés à la pluralité de sondes d'hybridation d'ADN dénaturées. Les fragments d'ADN hybridés à la pluralité de sondes d'hybridation d'ADN dénaturées ont ensuite été lavés à l'aide de deux étapes de lavage et récupérés pour former un milieu enrichi avec des séquences d'ADN cibles. La première étape de lavage est réalisée à une température de 25°C tandis que la deuxième étape de lavage est réalisée à une température de 48°C. Ensuite, les séquences d'ADN cibles ont été amplifiés par une deuxième amplification PCR à l'aide de 9 cycles d'amplification PCR pour donner un échantillon enrichi en des séquences d'ADN cibles à séquencer. Enfin, un contrôle qualité de l'échantillon enrichi en des séquences d'ADN cibles à séquencer a été réalisé où la concentration d'ADN de l'échantillon enrichi en des séquences d'ADN cibles à séquencer est de 16 ng/µl avec une taille moyenne des séquences d'ADN cibles à séquencer de 380 bp.

En réalisant ces différentes étapes de l'exemple 2, un enrichissement uniforme, défini par une uniformité supérieure à 90%, de fragments d'ADN contenant des séquences simples et de fragments d'ADN contenant des séquences complexes desdits fragments d'ADN hybridés à la pluralité de sondes d'hybridation d'ADN dénaturées a pu être obtenu et où un pourcentage de séquences dupliquées est inférieur à 16%.

### Exemple 3.- Séquençage ciblé des séquences d'ADN cibles à séquencer d'un échantillon d'une tumeur solide.

A partir de l'échantillon enrichi en des séquences d'ADN cibles à séquencer obtenu (voir exemple 2), un séquençage a été réalisé à l'aide d'un séquenceur NextSeq500 où la concentration de chargement des cellules d'écoulement est de 1,6 pM et où le séquençage réalisé est un séquençage à extrémités jumelés 'paired end sequencing' : 2^{∗}75 pb. La couverture moyenne de séquençage est de minimum 400. Ce séquençage a permis d'obtenir une densité des grappes (clusters) d'environ 265 000/mm² avec un filtre de passage des grappes (clusters) de 84,5% pour un nombre total de lecture de séquençage d'environ 228 000 000 (228 millions).

### Exemple 4.- Caractérisation des aberrations génétiques de l'échantillon d'une tumeur solide.

A l'aide d'une analyse bioinformatique, les données de séquençage ciblé obtenues (voir exemple 3) ont été comparées à une séquence de référence (hg19, grch37) de NCBI: https://www.ncbi.nlm.nih.aov/search/all/?term=arch37.p13.), ce qui a permis d'identifier les aberrations génétiques de la tumeur. Ensuite un rapport théranostique a été réalisé comprenant des informations médicales, des données du séquençage ciblé de la tumeur avec une liste des aberrations génétiques identifiées, un immunogramme définissant un potentiel de réponse à une immunothérapie basé sur la caractérisation de la tumeur, une liste de traitements avec leurs propriétés qui pourraient être associés à un bénéfice clinique et ceux qui ne seraient pas associés à un bénéfice, une liste des essais cliniques associés à la caractérisation de la tumeur et une liste des publications en lien avec la caractérisation de la tumeur.

Le temps total d'analyse comprenant la récolte d'ADN génomique et la préparation de cet ADN génomique (exemple 1), de l'enrichissement en fragments d'ADN contenant des séquences simples et en fragments d'ADN contenant des séquences complexes (exemple 2), du séquençage ciblé des séquences d'ADN cibles à séquencer (exemple 3) et de la caractérisation de la tumeur en identifiant des aberrations génétiques de la tumeur et en établissant un rapport théranostique de la tumeur (exemple 4 ci-dessus) est de 10 jours ouvrés.

### Exemple comparatif 1.- Comparaison d'un séquençage d'exomes

Le séquençage d'exomes exemplifié compare 2 méthodes :
1. une méthode selon la présente invention en utilisant des sondes d'hybridation d'ADN double brin pour exomes pour les étapes d'hybridation et d'enrichissement (voir protocole de la figure 1 et exemple 2), et
2. une méthode selon la technologie Agilent qui utilise un séquençage de nouvelle génération basé sur un enrichissement des cibles par une capture d'hybridation en utilisant des sondes pour exomes (SureSelect Focused Exome et SureSelect Human All Exome).

**Tableau 2: Caractéristiques de spécificité du séquençage selon la méthode selon la présente invention et selon la technologie Agilent.**

| | **Séquençage selon la présente invention** | **Agilent technologie** |
|---|---|---|
| **Appâts (séquences) couverts (couverture de séquençage)** | 36695332 | 65962670 |
| **Taille des séquences ciblées** | 33359393 | 45659296 |
| **Pourcentage d'appâts parmi les séquences cibles** | 90% | 70% |

Le Tableau 2 montre des caractéristiques de spécificité du séquençage selon les 2 méthodes explicitées ci-dessus. La proportion de la taille des séquences ciblées par rapport à la couverture de séquençage est supérieure (90% par rapport à 70%) dans la méthode selon la présente invention utilisant des sondes d'hybridation d'ADN double brin, ce qui démontre une plus grande spécificité du séquençage avec la méthode selon la présente invention. Cette plus grande spécificité permet de minimiser la profondeur de séquençage pour obtenir une quantité d'information donnée de séquençage, ce qui accélère et réduit les coûts de séquençage.

Concernant l'uniformité de séquençage, la méthode selon la présente invention, utilisant des sondes d'hybridation d'ADN double brin pour exomes, permet d'avoir une profondeur moyenne de séquençage de 70 fois où 99% des cibles ont été couvertes plus de 25 fois, 79% des cibles ont été couvertes plus de 50 fois et seulement 3% des cibles ont été couvertes plus de 100 fois. En comparaison, avec la technologie Agilent, la profondeur moyenne de séquençage est de 64 fois où 93% des cibles ont été couvertes plus de 25 fois, 66% des cibles ont été couvertes plus de 50 fois et 12% des cibles ont été couvertes plus de 100 fois. L'uniformité de la couverture de séquençage est donc plus grande en utilisant des sondes d'hybridation d'ADN double brin selon la présente invention.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Méthode de caractérisation d'une tumeur par un séquençage ciblé à partir d'un échantillon d'un patient atteint d'un cancer, comprenant les étapes de :
- une récolte d'ADN génomique à partir dudit échantillon comprenant au moins une cellule cancéreuse,
- une préparation d'une bibliothèque de séquençage à partir dudit échantillon comprenant les étapes successives de (i) une fragmentation dudit ADN génomique générant des fragments d'ADN, (ii) un ajout d'une série d'étiquettes aux extrémités desdits fragments d'ADN, (iii) une première amplification desdits fragments d'ADN à l'aide d'amorces ayant une séquence complémentaire à une séquence desdites étiquettes formant un premier mélange comprenant des fragments d'ADN amplifiés,
- un blocage desdits fragments d'ADN amplifiés dudit premier mélange à l'aide d'une série de bloqueurs ayant une séquence complémentaire à au moins une partie de la séquence des étiquettes pour former des fragments d'ADN bloqués, et
- un séquençage et une caractérisation de la tumeur,
**caractérisée en ce que** ledit séquençage et ladite caractérisation de la tumeur comprennent les étapes de :
- une collecte d'une pluralité de sondes d'hybridation d'ADN double brin spécifiques à une pluralité desdits fragments d'ADN bloqués,
- une dénaturation desdites sondes d'hybridation d'ADN double brin pour former une pluralité de sondes d'hybridation d'ADN dénaturées spécifiques à ladite pluralité desdits fragments d'ADN bloqués,
- une hybridation de ladite pluralité desdits fragments d'ADN bloqués par ladite pluralité de sondes d'hybridation d'ADN dénaturées pour former un deuxième mélange comprenant des fragments d'ADN hybridés et des fragments d'ADN non-hybridés à ladite pluralité de sondes d'hybridation d'ADN dénaturées,
- un enrichissement uniforme et simultané du deuxième mélange en fragments d'ADN contenant des séquences simples et en fragments d'ADN contenant des séquences complexes desdits fragments d'ADN hybridés à la pluralité de sondes d'hybridation d'ADN dénaturées par une capture desdits fragments d'ADN hybridés pour former un milieu enrichi en lesdits fragments d'ADN hybridés à la pluralité de sondes d'hybridation d'ADN dénaturées,
- un lavage et une récupération desdits fragments d'ADN hybridés à la pluralité de sondes d'hybridation d'ADN dénaturées pour former un milieu enrichi avec des séquences d'ADN cibles,
- une deuxième amplification desdites séquences d'ADN cibles à l'aide d'amorces ayant une séquence complémentaire à une séquence desdites étiquettes donnant des séquences d'ADN cibles à séquencer,
- ladite caractérisation de la tumeur étant une caractérisation par un séquençage des fragments d'ADN contenant des séquences simples et des fragments d'ADN contenant des séquences complexes desdites séquences d'ADN cibles à séquencer de manière à identifier des aberrations génétiques de la tumeur.

2. Méthode selon la revendication 1, dans laquelle lesdites séquences complexes desdites séquences d'ADN cibles à séquencer étant des séquences présentant un pourcentage de base nucléotidique guanine et cytosine égal ou supérieur à 60%, préférentiellement égal ou supérieur à 70%, de manière favorable égale ou supérieur à 80% ou des séquences répétitives ou des séquences inversées.

3. Méthode selon la revendication 1 ou la revendication 2, dans laquelle lesdits fragments d'ADN hybridés à ladite pluralité de sondes d'hybridation d'ADN dénaturées présentent une séquence dont au moins une partie est une séquence codante desdits fragments d'ADN contenant les séquences simples ou desdits fragments d'ADN contenant les séquences complexes et/ou une séquence dont au moins une partie est une séquence non-codante desdits fragments d'ADN contenant les séquences simples ou desdits fragments d'ADN contenant les séquences complexes .

4. Méthode selon l'une quelconque des revendications 1 à 3, dans laquelle lesdites séquences d'ADN cibles à séquencer contiennent des aberrations génétiques comprenant (i) une série de polymorphisme d'un seul nucléotide d'une ou de plusieurs séquences d'ADN par rapport à une ou plusieurs séquences d'ADN correspondante d'un génome de référence, ladite série de polymorphisme d'un seul nucléotide comprenant préférentiellement au moins 50, plus préférentiellement au moins 500, encore plus préférentiellement au moins 1500, de manière favorable au moins 3000 polymorphismes d'un seul nucléotide, lesdits polymorphismes d'un seul nucléotide étant localisés, en moyenne sur tout le génome, toutes les 0,5 à 50 mégabases, préférentiellement toutes les 5 à 25 mégabases, plus préférentiellement toutes les 10 à 20 mégabases, de manière favorable toutes les 14 à 15 mégabases, et/ou (ii) une délétion d'une copie ou de deux copies d'un gène par rapport audit génome de référence, et/ou (iii) une variation du nombre de copies par rapport au nombre de copies dudit génome de référence associée à au moins 2 régions génomiques, préférentiellement au moins 50 régions génomiques, plus préférentiellement au moins 100 régions génomiques, de manière favorable au moins 250 régions génomiques et à maximum 5000 régions génomiques, de manière préférée maximum 2000 régions génomiques, de manière favorable maximum 1000 régions génomiques réparties de manière aléatoire dans le génome, et/ou (iv) un phénotype de défaut de recombinaison homologue déterminé par comparaison d'au moins une desdites séquences d'ADN cibles à séquencer par rapport à au moins une séquence d'ADN correspondante dudit génome de référence, et/ou (v) une charge de mutation tumorale déterminée à l'aide d'une comparaison d'une pluralité de séquences codantes desdites séquences d'ADN cibles à séquencer avec une pluralité de séquences codantes correspondantes dudit génome de référence, ladite pluralité de séquences codantes desdites séquences d'ADN cibles à séquencer étant déterminée par un séquençage d'au moins 1 mégabase de séquences codantes, préférentiellement au moins 1,1, plus préférentiellement au moins 1,2, 1,3, 1,4, 1,5, 1,6, 1,7, 1,8, 1,9, 2 mégabases de séquences codantes desdites séquences d'ADN cibles à séquencer, et/ou (vi) une instabilité microsatellitaire déterminée à l'aide d'une comparaison d'une série desdites séquences d'ADN cibles à séquencer par rapport à une série de séquences d'ADN correspondante dudit génome de référence, ladite série desdites séquences d'ADN cibles à séquencer est déterminée par un séquençage, d'au moins une séquence d'ADN cibles à séquencer, préférentiellement au moins 100 séquences d'ADN cibles à séquencer, plus préférentiellement au moins 1000 séquences d'ADN cibles à séquencer, de manière favorable au moins 1500 séquences d'ADN cibles à séquencer.

5. Méthode selon l'une quelconque des revendications 1 à 4, dans laquelle ladite caractérisation de la tumeur comprend (i) un séquençage d'au moins une partie d'une séquence d'au moins 100, plus particulièrement d'au moins 200, encore plus particulièrement d'au moins 300, de préférence au moins 325, ou mieux, au moins, 350, 375, 400, 425, 450, 475, 500, 525, 550, 575, 600, plus particulièrement de chaque gène d'un panel de gènes listés au Tableau 1 ou d'un séquençage d'au moins une partie d'une séquence de chaque gène d'un sous-groupement de gènes compris dans le Tableau 1 pour identifier une signature d'aberrations génétiques associées à une thérapie, et/ou (ii) une identification d'une aberration génétique par rapport audit génome de référence d'au moins une séquence codante ou non-codante d'un gène associée à un traitement contre le cancer, et/ou d'au moins une séquence non-codante au sein d'une séquence d'un gène associée aux translocations associées à un traitement contre le cancer et/ou d'au moins une région d'épissage d'au moins un gène associée à un traitement contre le cancer, et/ou (iii) une identification d'une expression d'au moins un marqueur tumoral mesurée par son taux d'ARN et/ou de microARN et/ou de protéine.

6. Méthode selon l'une quelconque des revendications 1 à 5, dans laquelle lesdites séquences d'ADN cibles à séquencer étant **caractérisées par** des aberrations génétiques comprenant un déséquilibre allélique des télomères par rapport audit génome de référence identifié par au moins deux, préférentiellement au moins 100, plus préférentiellement au moins 1000, de manière favorable au moins 2000, et de préférence maximum 20000, plus préférentiellement maximum 10000, de manière favorable maximum 6000 polymorphismes d'un seul nucléotide et/ou insertions et/ou délétions d'un nucléotide d'au moins 1 fragment d'ADN localisé dans une région pré-télomérique.

7. Méthode selon la revendication 6, dans laquelle lesdits polymorphismes d'un seul nucléotide d'au moins 1 fragment d'ADN localisé dans une région pré-télomérique sont déterminés avec une fréquence des allèles mineurs égale ou supérieure à 20%, préférentiellement égale ou supérieure à 25%, de manière favorable égale ou supérieure à 30%.

8. Méthode selon l'une quelconque des revendications 1 à 7, dans laquelle lesdites séquences d'ADN cibles à séquencer étant **caractérisées par** des aberrations génétiques comprenant 1) un phénotype de défaut de recombinaison homologue (HRD), ledit phénotype HRD étant déterminé par (i) une identification d'une série de polymorphisme d'un seul nucléotide d'au moins une séquence d'ADN cible à séquencer par rapport à au moins une séquence d'ADN correspondante dudit génome de référence, ladite au moins une séquence d'ADN cible à séquencer étant localisée dans au moins une région génomique au sein d'un gène et, (ii) une identification d'une série de polymorphisme d'un seul nucléotide et/ou d'une série d'insertion et/ou d'une série de délétion d'un nucléotide par comparaison d'au moins une séquence d'ADN cible à séquencer localisée dans une région pré-télomérique par rapport à au moins une séquence d'ADN correspondante localisée dans une région pré-télomérique correspondante dudit génome de référence, ladite série de polymorphisme d'un seul nucléotide d'au moins une séquence d'ADN cible à séquencer localisée dans une région pré-télomérique est déterminée avec une fréquence des allèles mineurs égale ou supérieure à 20%, préférentiellement égale ou supérieure à 25%, de manière favorable égale ou supérieure à 30%, et/ou 2) au moins 2, préférentiellement au moins 3, plus préférentiellement au moins 4, encore plus préférentiellement au moins 5, de manière favorable au moins 6 aberrations génétiques choisies dans un groupe d'aberrations génétiques comprenant un polymorphisme d'un seul nucléotide, une délétion d'une copie ou de deux copies d'un gène, une variation du nombre de copies, un phénotype de défaut de recombinaison homologue, une charge de mutation tumorale, une instabilité microsatellitaire, un déséquilibre allélique des télomères, ou une mutation, une translocation ou un épissage associé à un traitement contre le cancer, lesdites aberrations génétiques étant déterminées par comparaison desdites séquences d'ADN cibles à séquencer par rapport aux séquences d'ADN correspondantes du génome de référence.

9. Méthode selon l'une quelconque des revendications 1 à 8, dans laquelle ladite pluralité de sondes d'hybridation d'ADN dénaturées spécifiques à ladite pluralité desdits fragments d'ADN bloqués est un mélange contenant plusieurs sondes d'hybridation de séquences identiques ou différentes mais complémentaires à au moins 60%, préférentiellement au moins 70%, plus préférentiellement au moins 80%, encore plus préférentiellement au moins 90%, de manière préférée au moins 95%, 96%, 97%, 98%, 99% de la séquence d'un ou de plusieurs fragments de la pluralité desdits fragments d'ADN bloqués, et où ladite pluralité de sondes d'hybridation d'ADN dénaturées spécifiques à ladite pluralité desdits fragments d'ADN bloqués est une sonde d'ADN dénaturée par fragment d'ADN bloqué ou plusieurs sondes d'ADN dénaturée par fragment d'ADN bloqué.

10. Méthode selon l'une quelconque des revendications 1 à 9, dans laquelle lesdites séquences d'ADN cibles à séquencer possèdent une séquence d'une longueur moyenne comprise entre 10 et 10000 paires de bases, préférentiellement entre 100 et 1000 paires de bases, plus préférentiellement entre 200 et 600 paires de bases, de manière favorable entre 375 et 425 paires de bases.

11. Méthode selon l'une quelconque des revendications 1 à 10 pour identifier un traitement basé sur ladite caractérisation de ladite tumeur.

12. Méthode selon l'une quelconque des revendications 1 à 11 comprenant en outre un établissement d'une cartographie de la tumeur dans laquelle les aberrations génétiques de la tumeur sont indiquées en regard de traitements recommandés pour les tumeurs **caractérisées par** ces aberrations génétiques, lesdits traitements recommandés étant obtenus par comparaison des aberrations génétiques de la tumeur analysée avec des aberrations génétiques de tumeurs de référence et de leurs traitements thérapeutiques de référence enregistrés dans une base de données.

13. Un agent ou plusieurs agents pour traiter une tumeur d'un patient dont ladite tumeur a été **caractérisée par** la méthode selon une quelconque des revendications précédentes 1 à 10, ledit agent ou plusieurs agents étant choisi dans le groupe comprenant des inhibiteurs PARP, des thérapies ciblées, des immunothérapies, des chimiothérapies, des radiothérapies, des agents endommageant l'ADN comme des agents à base de platine, des thérapies adjuvantes, des inhibiteurs de tyrosine kinases, des inhibiteurs du point de contrôle immunitaire, l'erlotinib, le gefitinib, l'afatinib, l'osimertinib, le dacomitinib, le cabozantinib, le crizotinib, l'alectinib, le ceritinib, le brigatinib, le vemurafenib, l'encorafenib, le dabrafenib, le trametinib, le cobimetinib, le docetaxel, le paclitaxel, le gemcitabine, le pemetrexed, le pembrolizumab, l'atezolizumab, le nivolumab, le durvalumab, l'olaparib, le niraparib, le talazoparib, le rucaparib, le trastuzumab, le pertuzumab, le neratinib, un traitement ou une thérapie approuvée ou en cours de développement.

14. Utilisation d'une pluralité de sondes d'hybridation d'ADN double brin pour réaliser un séquençage ciblé de fragments d'ADN contenant des séquences simples et de fragments d'ADN contenant des séquences complexes d'un échantillon d'un patient atteint d'un cancer, ledit échantillon comprenant au moins une cellule tumorale.

15. Rapport théranostique d'un patient atteint d'un cancer, obtenu en mettant en œuvre la méthode selon l'une quelconque des revendications 1 à 12, destiné à être utilisé pour déterminer un traitement ou une thérapie approuvée ou en cours de développement du cancer du patient.
